(19) Europäisches Patentamt
European Patent Office
Office européen des brevets

(11) **EP 1 317 439 B1**

(12) **FASCICULE DE BREVET EUROPEEN**

(45) Date de publication et mention de la délivrance du brevet:
**07.12.2005 Bulletin 2005/49**

(21) Numéro de dépôt: **01967418.3**

(22) Date de dépôt: **03.09.2001**

(51) Int Cl.⁷: **C07D 317/34**, C07D 319/06, C07J 41/00, A61K 47/48, A61P 9/10

(86) Numéro de dépôt international:
**PCT/FR2001/002725**

(87) Numéro de publication internationale:
**WO 2002/020510 (14.03.2002 Gazette 2002/11)**

(54) **COMPOSES ACIDOSENSIBLES, LEUR PREPARATION ET UTILISATIONS**

SÄUREEMPFINDLICHE VERBINDUNGEN, DEREN HERSTELLUNG UND VERWENDUNGEN

ACID-SENSITIVE COMPOUNDS, PREPARATION AND USES THEREOF

(84) Etats contractants désignés:
**AT BE CH CY DE DK ES FI FR GB GR IE IT LI LU MC NL PT SE TR**
Etats d'extension désignés:
**AL LT LV MK RO SI**

(30) Priorité: **05.09.2000 FR 0011278**
**11.10.2000 US 239116 P**

(43) Date de publication de la demande:
**11.06.2003 Bulletin 2003/24**

(73) Titulaire: **CENTELION**
**94400 Vitry Sur Seine (FR)**

(72) Inventeurs:
- **BESSODES, Michel**
  **F-94800 VILLEJUIF (FR)**
- **MASSON, Christophe**
  **F-91230 MONTGERON (FR)**
- **SCHERMAN, Daniel**
  **F-75012 PARIS (FR)**
- **WETZER, Barbara**
  **F-75005 PARIS (FR)**

(74) Mandataire: **Peaucelle, Chantal et al**
**Cabinet Armengaud Ainé**
**3, avenue Bugeaud**
**75116 Paris (FR)**

(56) Documents cités:
**US-A- 4 406 686** **US-A- 6 013 240**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 1 317 439 B1

Printed by Jouve, 75001 PARIS (FR)

## Description

La présente invention concerne des composés acidosensibles et leur préparation. Ces composés comprennent au moins un substituant hydrophile et un orthoester cyclique qui est acidosensible. Ces composés sont utiles soit pour former des conjugués (liposomes, complexes, nanoparticules ...) avec des substances biologiquement actives et les libérer dans des tissus ou compartiments cellulaires dont le pH est acide, soit en tant qu'agent de surface non-ionique pour stabiliser des particules encapsulant une substance biologiquement active puis les déstabiliser en milieu acide, ou encore en tant que vecteur lié covalemment à une molécule thérapeutique afin de la libérer dans les tissus ou compartiments cellulaires dont le pH est acide.

La libération de substances biologiquement actives dans des tissus ou des cellules présentant une acidité accentuée par rapport à la normale physiologique est un problème connu qui a fait l'objet de nombreuses études, sans pour autant donner de résultats complètement satisfaisants jusqu'à présent. Ainsi, de nombreux liposomes pH-sensibles ont été conçus afin de libérer des substances biologiquement actives en tirant avantage de l'acidification de certains tissus ou de l'endosome.

Par exemple, Yatvin et al. (*Science,* Vol. 210, 1980, pp. 1253-4) a conçu des lipides pH-sensibles, capables de s'insérer dans la bicouche lipidique de liposomes classiques, de formule :

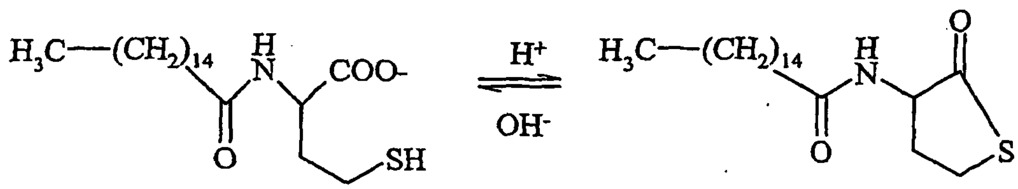

L'homocystéine présente dans ce lipide est sous sa forme ouverte à pH neutre ou alcalin et ressemble alors à un acide gras qui s'insère parfaitement dans la bicouche des liposomes. A pH acide, elle se présente sous sa forme fermée : elle forme alors une thiolactone cyclique, ressemblant ainsi à un lipide neutre qui déstabilise la bicouche liposomale et permet ainsi la libération de la substance active. Une telle molécule permet la libération de molécules médicamenteuses dans les régions du corps dans lesquelles le pH est inférieur au pH physiologique, par exemple au niveau des tumeurs primaires, des métastases, ou encore des sites d'inflammation et d'infection.

Le brevet américain US 5,965,434 propose des lipides amphiphatiques comprenant une partie hydrophile cationique et pH-sensible de formule :

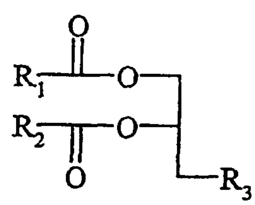

dans laquelle $R_1$ et $R_2$ représentent indépendamment l'un de l'autre $CH_3(CH_2)_{14}$, $CH_3(CH_2)_{12}$, $CH_3(CH_2)_7CHCH(CH_2)_7$, et $R_3$ représente un substituant 1-méthylimidazole, imidazole, 4,9-dioxo-1,12-dodécanediamine, cystéamine, 1-(3-aminopropyl) imidazole, morpholine, 4-aminopyridine, pyridine, guanidine, hydrazine, thiouronium ou pipérazine. Ces composés présentent la particularité de porter une charge positive globale (au niveau du composé $R_3$) qui augmente lorsque le pH diminue de 8,0 à 4,5. Cette modification de la charge induit une transformation conformationnelle du liposome lui permettant de relarguer son contenu. Ces lipides permettent ainsi la libération de molécules médicamenteuses ou d'acides nucléiques dans des milieux acide dont le pH varie jusqu'à 4,5.

Par ailleurs, la demande WO 97/31624 propose des phospholipides pH-sensibles (« triggerable lipids » ) qui comprennent une fonction éther vinylique qui peut être dégradée dans le cytoplasme, et qui ont pour formule générale :

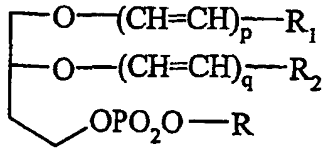

dans laquelle p et q valent 0 ou 1, l'un au moins des deux étant égal à 1, $R_1$ et $R_2$ représentent indépendamment l'un de l'autre un alkyle ou un alcène contenant 12 à 24 atomes de carbone, et R représente un groupe choisi parmi le 2-aminoéthyle, 2-(triméthylamino)éthyle, 2-(N,N-diméthylamino)éthyle, 2-(triméthylammonium) éthyle, 2-carboxy-2-aminoéthyle, succinamidoéthyle ou l'inositoyl.

Ces phospholipides sont mélangés à d'autres phospholipides eux-mêmes complexés à des ligands de récepteurs cellulaires, afin de former des liposomes capables de subir des changements conformationnels à pH acide. De tels liposomes permettent l'encapsulation de nombreuses substances médicamenteuses et aussi d'acides nucléiques pour la thérapie génique.

Une autre approche a également été décrite (*Kratz et al.*, Crit. Rev. Ther. Drug Carrier Syst. 1999, 16(3), pp. 245-88), dans laquelle une molécule thérapeutique est liée covalemment à un polymère par l'intermédiaire d'une liaison acidosensible de façon à assurer la libération de ladite molécule thérapeutique dans les tissus tumoraux faiblement acides ou bien dans les endosomes et lysosomes après internalisation cellulaire du conjugué polymérique. De nombreuses liaisons possibles ont ainsi été décrites, par exemple les liaisons acétale, disulfure, hydrazone, cis-aconitrile, trityle ou encore éther silylé.

Cependant, tous les composés pH-sensibles mis au point jusqu'à présent présentent l'inconvénient de ne pas être modulables en ce qui concerne leur sensibilité. Ainsi, il serait très intéressant de pouvoir disposer de composés acidolabiles dont la sensibilité pourrait être modulée en fonction par exemple des tissus ou cellules ciblées, de la substance biologiquement active à libérer ou encore des applications envisagées. De façon plus générale, il serait en outre intéressant de pouvoir disposer de nouveaux composés pH-sensibles qui soit faciles à préparer et efficaces pour la transfection d'acides nucléiques en particulier.

Afin de résoudre ce problème, la Demanderesse a ainsi mis au point une nouvelle famille de composés acidosensibles caractérisés en ce qu'ils comprennent un orthoester cyclique et au moins un substituant hydrophile choisi parmi les polyalkylènes glycols, les mono- ou polysaccharides, les molécules thérapeutiques hydrophiles, ou les radicaux de type polyamine.

De tels composés sont utiles pour la vectorisation et la libération de substances biologiquement actives dans des régions acides de l'organisme grâce à la fonction orthoester cyclique qui est acidosensible. Ils sont tout particulièrement intéressant car la sensibilité au pH du composé peut être modulée en fonction du choix du substituant présent sur le carbone central et de la taille du cycle orthoester. Il est ainsi possible de faire largement varier la cinétique d'hydrolyse de ces composés et donc de moduler le temps nécessaire à la libération de la substance biologiquement active. En outre, les composés acidosensibles selon l'invention présentent l'avantage supplémentaire de se dégrader en milieu acide de façon autocatalytique. En effet, la dégradation partielle des composés acidosensibles selon l'invention entraîne la libération progressive d'un acide (par exemple l'acide formique lorsque le composé de départ dérive d'un orthoformate, ou bien l'acide acétique lorsque le composé de départ dérive d'un orthoacétate, ou encore l'acide benzoïque lorsque le composé de départ dérive d'un orthobenzoate) qui induit une baisse du pH favorisant encore davantage leur dégradation.

Plus particulièrement, les composés acidosensibles selon la présente invention ont pour formule générale :

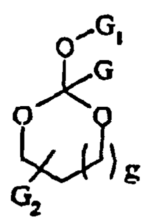

(I)

dans laquelle :

- g est un entier pouvant prendre les valeurs 0, 1, 2, 3 ou 4,
- G représente un atome d'hydrogène, un radical alkyl contenant 1 à 6 atomes de carbone, saturé on insaturé, en

chaîne droite ou ramifiée, ou un radical aryle contenant 6 à 14 atomes de carbone,

- $G_1$ et $G_2$ représentent :

(a) l'un un substituant hydrophile choisi parmi les radicaux de type polyamine, et l'autre un substituant hydrophobe choisi parmi les alkyles mono- ou bicaténaires, les dérivés de stéroïde ou les dendrimères hydrophobes, ou bien

(b) l'un un groupe alkyle linéaire hydrophobe comprenant 10 à 24 atomes de carbones et comprenant éventuellement une ou plusieurs insaturations, et l'autre on groupe de formule générale :

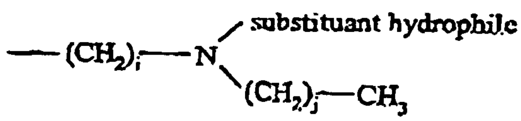

(II)

dans laquelle i est un entier choisi entre 1 et 4 inclus et j est un entier choisi entre 9 et 23 inclus, et le substituant hydrophile est choisi parmi les radicaux de type polyamine, ou bien

(c) l'un un substituant hydrophile choisi parmi les polyalkylène glycols ou les mono- ou polysaccharides et l'autre un substituant choisi parmi les polyalkylène imines, ou bien

(d) l'un un substituant hydrophile choisi parmi les polyalkylène glycols ou les mono- ou polysaccharides et l'autre un substituant hydrophobe choisi parmi les alkyles mono- ou bicaténaires, les dérivés de stéroïde, les dendrimères hydrophobes, ou les conjugués covalents entre un alkyle mono- ou bicaténaire, un dérivé de stéroïde, ou un dendrimère hydrophobe et une molécule de polyalkylène glycol comprenant 1 à 20 unités monomériques, ou bien

(e) l'un un substituant hydrophile choisi parmi les polyalkylène glycols ou les mono- ou polysaccharides et l'autre une molécule thérapeutique, ou bien

(f) l'un une molécule thérapeutique de nature hydrophile et l'autre un substituant hydrophobe choisi parmi les alkyles mono- ou bicaténaires, les dérivés de stéroïde ou les dendrimères hydrophobes.

Le substituant G placé sur le carbone central de l'orthoester est choisi de façon à moduler la sensibilité du composé acidosensible selon la présente invention. Ainsi, plus le groupe G sera électrodonneur, plus l'acidosensibilité du composé sera grande, et plus le groupe G sera électroattracteur, plus l'acidosensibilité du composé sera faible.Il apparaît ainsi que le choix du radical G est particulièrement important pour la détermination et la modulation des propriétés des composés acidosensibles de formule générale (I). Selon un aspect préféré de l'invention, G est choisi parmi l'atome d'hydrogène, les radicaux alkyles comprenant 1 à 6 atomes de carbone en chaîne droite ou ramifiée, saturée ou insaturée, ou les radicaux aryles. De façon particulièrement avantageuse, G est choisi parmi l'hydrogène, le méthyle, l'éthyle ou le phényle.

Au sens de la présente invention, on entend par « radicaux aryles » les radicaux hydrocarbonés aromatiques univalents. Les radicaux aryles selon la présente invention contiennent généralement entre 6 et 14 atomes de carbone. De preference, les radicaux aryles selon la présente invention sont choisis parmi le phényle, le naphthyle, par exemple 1-naphthyle ou 2-naphthyle, le biphénylyle, par exemple 2-biphénylyle, 3-biphénylyle ou 4-biphénylyle, l'anthryle ou le fluorényle. Le phényle est plus particulièrement préféré. Les radicaux aryles, en particulier le phényle, peuvent être substitués ou non, par exemple monosubstitués, disubstitués, trisubstitués ou tétrasubstitués, les substituants étant identiques ou différents. De préférence, lesdits substituants sont choisis parmi les atomes d'halogène, les radicaux alkyle ($C_1$-$C_8$) ou alkoxy ($C_1$-$C_8$). Dans le cas de radicaux phényles monosubstitués, ledit substituant peut être situé en position -2, en position -3 ou en position -4. Dans le cas de radicaux phényles disubstitués, lesdits substituants peuvent être situés en position -2,3, en position 2,4, en position -2,5, en position -2,6, en position -3,4 ou en position -3,5. Dans le cas de radicaux phényles trisubstitués, lesdits substituants peuvent être situés par exemple en position 2,3,4, en position -2,3,5, en position -2,4,5, en position -2,4,6, en position -2,3,6 ou en position -3,4,5.

Selon les cas, chacun des substituants $G_1$ et $G_2$ est soit directement lié à l'orthoester cyclique, soit indirectement par l'intermédiaire d'une molécule « espaceur » choisie parmi celles connues de l'homme de l'art. Une telle molécule espaceur permet tout à la fois d'assurer la liaison et d'éloigner le(s) substituant(s) concerné(s) de l'orthoester cyclique afin d'atténuer toute interaction non désirée entre l'orthoester cyclique acidosensible et son(ses) substituant(s). Des molécules espaceur préférées peuvent par exemple être choisies selon la nature des substituants $G_1$ ou $G_2$ parmi les alkyles (1 à 6 atomes de carbone), les liaisons carbonyles, esters, éthers, amide, carbamate, thiocarbamate, le glycérol, l'urée, la thiourée, ou une combinaison de plusieurs de ces groupes. Par exemple, lorsque le substituant hydrophobe est un dérivé de stéroïde, la molécule espaceur peut être une liaison de type carbamate -N-C(O)-O-, ou bien lorsque le substituant hydrophobe est un alkyle bicaténaire, la molécule espaceur peut être choisie parmi les

groupes de formule -alkyl-C(O)-N, les deux chaînes alkyle étant alors fixées sur l'atome d'azote.

Selon un aspect préféré de l'invention, les radicaux de type polyamine peuvent être définis comme étant des alkyles linéaires ou ramifiés comprenant au moins 3 atomes de carbone et dont l'un au moins des groupes méthylène peut être remplacé par un groupe amino éventuellement substitué (par un groupe méthyle par exemple) et le ou les méthyle(s) terminal(aux) est(sont) substitué(s) par un(des) groupe(s) choisi(s) parmi les amines (primaires, secondaires, tertiaires ou quaternaires), les guanidines ou les guanidines cycliques. De préférence, ces radicaux de type polyamine sont choisis parmi les radicaux polyaminés déjà connus et décrits dans la littérature pour la vectorisation d'acides nucléiques, par exemple dans les publications WO 96/17823, WO 97/18185, WO 98/54130 ou encore WO 99/51581. En particulier, il peut s'agir par exemple des polyamines de formule générale :

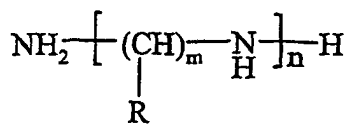

dans laquelle

- R représente un atome d'hydrogène à l'exception de un seul des groupes R qui est absent et représente donc la liaison covalente à l'orthoester cyclique
- n est un nombre entier compris entre 1 et 9 inclusivement
- m est un nombre entier compris entre 2 et 6 inclusivement, les valeurs de m pouvant être identiques ou différentes au sein des différents groupes $-(CH)_m-NH-$.

Selon une autre alternative, il peut aussi s'agir d'un radical de type polyamine de formule générale:

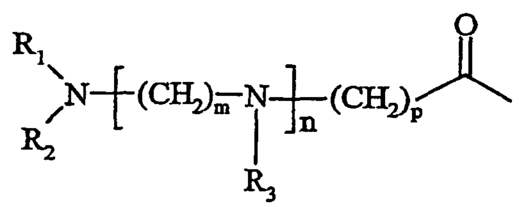

dans laquelle:

- $R_1$, $R_2$ et $R_3$ représentent indépendamment l'un de l'autre un atome d'hydrogène ou un groupement $-(CH_2)_q-NRR'$ avec q pouvant varier entre 1, 2, 3, 4, 5 et 6 ceci de manière indépendante entre les différents groupements $R_1$, $R_2$ et $R_3$ , et R et R' représentant indépendamment l'un de l'autre un atome d'hydrogène ou un groupement $-(CH_2)_{q'}-NH_2$, q' pouvant varier entre 1, 2, 3, 4, 5 et 6 ceci de manière indépendante entre les différents groupements R et R',
- m et p représentent, indépendamment l'un de l'autre, un nombre entier pouvant varier entre 1 et 6, et
- n représente un entier pouvant varier entre 0 et 6, avec lorsque n est supérieur à 1, m pouvant prendre des valeurs différentes et $R_3$ des significations différentes au sein de la formule générale, et avec lorsque n est égal à 0, l'un au moins de $R_1$ et de $R_2$ qui est différent de l'hydrogène.

Selon un autre aspect de la présente invention, le radical de type polyamine peut aussi être représenté par un substituant de formule générale identique à la précédente, mais avec R et R' représentant indépendamment l'un de l'autre un atome d'hydrogène ou un groupement de formule (1):

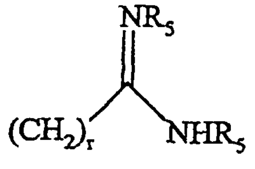

(1)

dans laquelle r est un nombre entier pouvant varier de 0 à 6 inclus, et les groupements $R_5$ représentent, indépendamment l'un de l'autre, un atome d'hydrogène, un substituant alkyle, carbamate ou acyle aliphatique ou aromatique, éventuellement halogéné, étant entendu que l'un au moins des groupes $R_1$, $R_2$ et $R_3$ comporte au moins un groupement de formule (1).

On obtient ainsi un radical de type polyamine comportant une ou des fonctions guanidine(s) terminale(s).

Selon une autre variante de l'invention, le radical de type polyamine peut également représenter une polyamine telle que celles décrites ci-avant mais avec un groupe terminal du type guanidine cyclique (au lieu d'une amine ou d'une guanidine) de formule générale (2) :

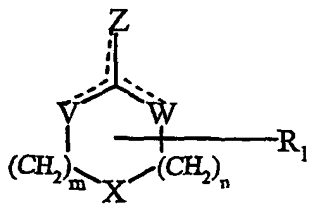

(2)

pour laquelle :

- m, et n sont des entiers indépendants l'un de l'autre compris entre 0 et 3 inclus et tels que m + n est supérieur ou égal à 1,
- $R_1$ représente un groupement de formule générale (3) :

$$-[(CH_2)_p-Y]_q-(*) \qquad (3)$$

  pour laquelle p et q sont des entiers indépendants l'un de l'autre compris entre 0 et 10 inclus, Y représente un groupement carbonyle, amino, méthylamino, ou bien méthylène, Y pouvant avoir des significations différentes au sein des différents groupements $[(CH_2)_p-Y]$, et (*) représente soit un atome d'hydrogène, soit une liaison covalente, étant entendu que $R_1$ peut être lié à n'importe quel atome de la formule générale (2), y compris Z, et qu'il y a un unique groupe $R_1$ dans la formule (2),
- X représente un groupement $NR_2$ ou bien $CHR_2$, $R_2$ étant soit un atome d'hydrogène soit la liaison au groupe $R_1$ tel que défini précédemment,
- Le groupement

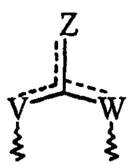

  représente :

  * 1^er^ cas : un groupement de formule générale (4) :

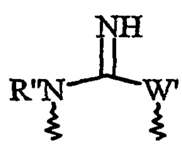

(4)

  pour laquelle W' représente CHR''' ou bien NR''', et R'' et R''' représentent indépendamment l'un de l'autre un atome d'hydrogène, un méthyle, ou la liaison au groupe $R_1$ tel que défini précédemment, ou bien

* 2ème cas : un groupement de formule générale (5) :

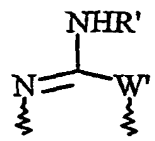

(5)

pour laquelle W' représente CHR''' ou bien NR''', et R' et R''' représentent indépendamment l'un de l'autre un atome d'hydrogène, un méthyle, ou la liaison au groupe $R_1$ tel que défini précédemment.

D'une manière générale, tout autre radical de type polyamine connu de l'homme de l'art pour s'associer aux acides nucléiques, notamment par l'intermédiaire d'interactions électrostatiques, peut également convenir.

Au sens de la présente invention, on entend par alkyles mono- ou bicaténaires les radicaux hydrophobes constitués d'une ou deux chaînes alkyles linéaires comprenant 10 à 24 atomes de carbones et comprenant éventuellement une ou plusieurs insaturations. Dans le cas des alkyles bicaténaires, il peut s'agir par exemple de deux chaînes alkyles liées à un atome d'azote de façon à former un substituant dialkylamino, les deux chaînes alkyles étant par exemple linéaires et compxennant 10 à 24 atomes de carbones et éventuellement une ou plusieurs insaturations. Il peut également s'agir d'acides gras saturés ou insaturés comme par exemple l'acide palmitique, l'acide oléique, l'acide stéarique, ou encore l'acide myristique. De préférence, les alkyles mono- ou bicaténaires possèdent 12 à 18 atomes de carbones, et encore plus préférentiellement, ils sont choisis parmi les groupes possédant 12, 14, 16 ou 18 atomes de carbone (pour chaque chaîne alkyle).

On entend par « dérivé de stéroïde» au sens de la présente invention les substituants choisi par exemple parmi les stérols, les stéroïdes et les hormones stéroïdiennes. Plus préférentiellement, les dérivés de stéroïde sont choisis parmi le cholestérol, le cholestanol, le 3-α-5-cyclo-5-α-cholestan-6-β-ol, l'acide cholique, le cholestérylformiate, le chotestanylformiate, le 3α,5-cyclo-5α-cholestan-6β-yl formiate, la cholestérylamine, la 6-(1,5-diméthylhexyl)-3a,5a-diméthylhexadécahydrocyclopenta[a] cyclopropa[2,3]cyclopenta[1,2-f]naphta-lèn-10-ylamine, la cholestanylamine ou encore le dexaméthasone.

Les dendrimères hydrophobes selon de la présente invention sont choisis de préférence parmi les poly(alkyl éther) hydrophobes ou encore les poly(aryl éther) hydrophobes. De façon particulièrement avantageuse, les dendrimères hydrophobes selon de la présente invention sont choisis parmi les poly(benzyl éther).

Au sens de la présente invention, les polyalkylène glycols sont préférentiellement choisis parmi les polyalkylènes glycols de poids moléculaire moyen compris entre $10^2$ et $10^5$ Daltons (Da), et éventuellement lié covalemment à un élément de ciblage. De façon particulièrement avantageuse, les polyalkylène glycols selon la présente invention sont choisis parmi les polyéthylène glycols (PEG) de poids moléculaire moyen compris entre $10^2$ et $10^5$ Da, et plus préférentiellement entre 500 et $10^5$ Da.

Au sens de la présente invention, on entend par « mono- ou polysaccharide » les molécules constituées de un ou plusieurs saccharides, éventuellement liés covalemment à un élément de ciblage. On peut citer à titre d'exemple les pyranoses et les furanoses, par exemple le glucose, le mannose, le rhamnose, le galactose, le fructose, ou encore le maltose, le lactose, le saccharose, le sucrose, le fucose, le cellobiose, l'allose, le laminarobiose, le gentiobiose, le sophorose, le mélibiose, etc... De plus, il peut également s'agir de saccharides dits « complexes », c'est-à-dire de plusieurs saccharrides couplés covalemment les uns aux autres, chaque sucre étant de préférence choisi dans la liste citée ci-avant. A titre de polysaccharides convenables, on peut citer les dextrans, l'α-amylose, l'amylopectine, les fructans, les mannans, les xylans et les arabinans. Préférentiellement, les mono- ou polysaccharides selon la présente invention sont choisis parmi les dérivés naturels ou commerciaux qui sont compatibles avec des applications pharmacologiques tels que les sucres naturels, les cyclodextrines ou encore les dextrans.

Selon une autre alternative de la présente invention, le polyalkylène glycol ou le mono- ou polysaccharide peuvent être éventuellement lié covalemment à un élément de ciblage. Dans ce cas, il peut s'agir soit d'un élément de ciblage extracellulaire permettant d'orienter les composés acidosensibles selon la présente invention ou les compositions les contenant vers certains types cellulaires ou certains tissus souhaités (cellules tumorales, cellules hépatiques, cellules hématopoiétiques...), ou bien il peut s'agir d'un élément de ciblage intracellulaire permettant une orientation vers certains compartiments cellulaires privilégiés (mitochondries, noyau etc...).

Parmi les éléments de ciblage utilisables dans le cadre de l'invention, on peut citer les sucres, les peptides, les protéines, les oligonucléotides, les lipides, les neuromédiateurs, les hormones, les vitamines ou leurs dérivés. Préférentiellement, il s'agit de sucres, de peptides, de vitamines ou de protéines tels que par exemple des anticorps ou des fragments d'anticorps, des ligands de récepteurs cellulaires ou des fragments de ceux-ci, des récepteurs ou encore des fragments de récepteurs. Par exemple, il peut s'agir de ligands de récepteurs de facteurs de croissance, de récepteurs de cytokines, de récepteurs de type lectines cellulaires, de récepteurs au folate, ou de ligands à séquence

RGD avec une affinité pour les récepteurs de protéines d'adhésion comme les intégrines. On peut également citer les récepteurs de la transferrine, des HDL et des LDL, ou le transporteur du folate. L'élément de ciblage peut également être un sucre permettant de cibler des lectines tels que les récepteurs aux asialoglycoprotéines ou aux syalydés tel que le Sialyl Lewis X, ou encore un fragment Fab d'anticorps, ou un anticorps simple chaîne (ScFv).

Au sens de la présente invention, on entend par « polyalkylène imines » les polymères décrits dans la publication WO 96/02655, à savoir les polymères comprenant les unités monomériques de formule générale :

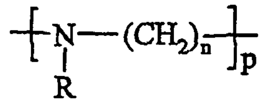

dans laquelle R peut être un atome d'hydrogène ou un groupe de formule :

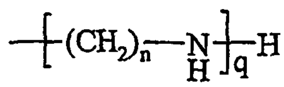

et n est un nombre entier compris entre 2 et 10, p et q sont des nombres entiers choisis de telle sorte que la somme p + q est telle que le poids moléculaire moyen du polymère soit compris entre 100 et $10^7$ Da.
Il est entendu que, dans cette formule, la valeur de n peut varier entre les différents motifs $-NR-(CH_2)_n-$. Ainsi, cette formule regroupe à la fois les homopolymères et les hétéropolymères. Les polyalkylèneimines commerciaux constituent une alternative intéressante. Les polyéthylèneimines (PEI) sont tout particulièrement préférés, et plus spécifiquement le PEI 25K (PEI de poids moléculaire moyen de 25 KDa), le PEI 50K, le PEI 100K ou encore le PEI 200K.

Selon la présente invention, on entend par « molécule thérapeutique » les molécules qui permettent de prévenir ou guérir une pathologie se manifestant dans des régions de l'organisme produisant une acidité accentuée par rapport à la normale physiologique. De telles régions sont plus spécifiquement, mais pas uniquement :

- les tumeurs, en particulier les cellules tumorales et aussi les cellules normales au voisinage de ces tumeurs (par exemple les cellules endothéliales des tumeurs), qui présentent une acidité locale plus importante que la normale physiologique (N. Raghunand et al., *Drug Resistance Updates,* 2000, 3, pp. 30-38),
- les muscles ischémiés, par exemple le muscle cardiaque, au niveau desquels l'acidose provient en partie de l'acide lactique produit par la fermentation anaérobie des hydrocarbones de type sucre ou des acides gras,
- les zones d'inflammation où la production d'ions superoxydes par les macrophages consomme beaucoup d'oxygène,
- ou encore les tissus où un trouble métabolique, infectieux ou inflammatoire produit une acidose locale.

Selon une autre alternative, les « molécule thérapeutique » selon la présente invention permettent de prévenir ou guérir une pathologie par leur libération dans un compartiment cellulaire acide, par exemple dans l'endosome des cellules qui est acide.

Les molécules thérapeutiques peuvent ainsi être choisies par exemple parmi les peptides, les oligopeptides, les protéines, les antigènes et leurs anticorps, les enzymes et leurs inhibiteurs, les hormones, les antibiotiques, les analgésiques, les bronchodilatateurs, les antimicrobiens, les agents antihypertenseurs, les agents cardiovasculaires, les agents agissants sur le système nerveux central, les antihistaminiques, les antidépresseurs, les tranquilisants, les anticonvulsifs, les substances anti-inflammatoires, les stimulants, les antiémétiques, les diurétiques, les antispasmodiques, les antiischémiques, les agents limitant la mort cellulaire, ou encore les agents anticancéreux.

En outre, on entend par « substance biologiquement active » les substances choisies soit parmi les molécules thérapeutiques telles que définies ci-avant, soit parmi les acides nucléiques.

On entend au sens de l'invention par « acide nucléique » aussi bien un acide désoxyribonucléique qu'un acide ribonucléique. Il peut s'agir de séquences naturelles
ou artificielles, et notamment d'ADN génomique (ADNg), d'ADN complémentaire (ADNc), d'ARN messager (ARNm), d'ARN de transfert (ARNt), d'ARN ribosomique (ARNr), de séquences hybrides ou de séquences synthétiques ou semi-synthétiques, d'oligonucléotides modifiés ou non. Ces acides nucléiques peuvent être par exemple d'origine humaine, animale, végétale, bactérienne, virale ou encore synthétique. Ils peuvent être obtenus par toute technique

connue de l'homme du métier, et notamment par criblage de banques, par synthèse chimique, ou encore par des méthodes mixtes incluant la modification chimique ou enzymatique de séquences obtenues par criblage de banques. Ils peuvent être modifiés chimiquement.

Concernant plus particulièrement les acides désoxyribonucléiques, ils peuvent être simple ou double brin de même que des oligonucléotides courts ou des séquences plus longues. En particulier, les acides nucléiques sont avantageusement constitués par exemple par des plasmides, des vecteurs, des épisomes ou des cassettes d'expression. Ces acides désoxyribonucléiques peuvent notamment porter une origine de réplication fonctionnelle ou non dans la cellule cible, un ou plusieurs gènes marqueurs, des séquences régulatrices de la transcription ou de la réplication, des gènes d'intérêt thérapeutique, des séquences antisens modifiées ou non, ou encore des régions de liaison à d'autres composants cellulaires.

De préférence, l'acide nucléique comprend une cassette d'expression constituée d'un ou plusieurs gènes d'intérêt thérapeutique sous contrôle d'un ou plusieurs promoteurs et d'un terminateur transcriptionnel actifs dans les cellules cibles.

On entend au sens de l'invention par « cassette d'expression d'un gène d'intérêt » un fragment d'ADN qui peut être inséré dans un vecteur à des sites de restriction spécifiques. Le fragment d'ADN comprend une séquence d'acide nucléique codant pour un ARN ou un polypeptide d'intérêt et comprend en outre les séquences nécessaires à l'expression (activateur(s), promoteur(s), séquences de polyadénylation etc...) de ladite séquence. La cassette et les sites de restriction sont conçus pour assurer une insertion de la cassette d'expression dans un cadre de lecture approprié pour la transcription et la traduction.

Il s'agit généralement d'un plasmide ou d'un épisome portant un ou plusieurs gènes d'intérêt thérapeutique. A titre d'exemple on peut citer les plasmides décrits dans les demandes de brevet WO 96/26270 et WO 97/10343 incorporées à la présente par référence.

Au sens de l'invention, on entend par gène d'intérêt thérapeutique notamment tout gène codant pour un produit protéique ayant un effet thérapeutique. Le produit protéique ainsi codé peut être notamment une protéine ou un peptide. Ce produit protéique peut être exogène homologue ou endogène vis-à-vis de la cellule cible, c'est-à-dire un produit qui est normalement exprimé dans la cellule cible lorsque celle-ci ne présente aucune pathologie. Dans ce cas, l'expression d'une protéine permet par exemple de pallier une expression insuffsante dans la cellule ou l'expression d'une protéine inactive ou faiblement active en raison d'une modification, ou encore de surexprimer ladite protéine. Le gène d'intérêt thérapeutique peut aussi coder pour un mutant d'une protéine cellulaire, ayant par exemple une stabilité accrue ou une activité modifiée. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire.

Parmi les produits thérapeutiques au sens de la présente invention, on peut citer plus particulièrement les enzymes, les dérivés sanguins, les hormones, les lymphokines (les interleukines, interférons ou le TNF par exemple : FR 92/03120), les facteurs de croissance, les neurotransmetteurs ou leurs précurseurs ou enzymes de synthèse, les facteurs trophiques (par exemple BDNF, CNTF, NGF, IGF, GMF, aFGF, bFGF, NT3, NT5, ou encore HARP/pléiotrophine) les apolipoprotéines (par exemple ApoAI, ApoAIV ou ApoE : FR 93/05125), la dystrophine ou une minidystrophine (FR 91/11947), la protéine CFTR associée à la mucoviscidose, les gènes suppresseurs de tumeurs (par exemple p53, Rb, Rap1A, DCC, ou k-rev : FR 93/04745), les gènes codant pour des facteurs impliqués dans la coagulation (Facteurs VII, VIII, IX), les gènes intervenant dans la réparation de l'ADN, les gènes suicides (thymidine kinase, cytosine déaminase), les gènes de l'hémoglobine ou d'autres transporteurs protéiques, les enzymes du métabolisme, catabolisme etc...

L'acide nucléique d'intérêt thérapeutique peut également être un gène ou une séquence antisens, dont l'expression dans la cellule cible permet de contrôler l'expression de gènes ou la transcription d'ARNm cellulaires. De telles séquences peuvent, par exemple, être transcrites dans la cellule cible en ARN complémentaires d'ARNm cellulaires et bloquer ainsi leur traduction en protéine, selon la technique décrite dans le brevet EP 140 308. Les gènes thérapeutiques comprennent également les séquences codant pour des ribozymes, qui sont capables de détruire sélectivement des ARN cibles (EP 321 201).

Comme indiqué plus haut, l'acide nucléique peut également comporter un ou plusieurs gènes codant pour un peptide antigénique, capable de générer chez l'homme ou l'animal une réponse immunitaire. Dans ce mode particulier de mise en oeuvre, l'invention permet la réalisation soit de vaccins soit de traitements immunothérapeutiques appliqués à l'homme ou à l'animal, notamment contre des microorganismes, des virus ou des cancers. Il peut s'agir notamment de peptides antigéniques spécifiques du virus d'Epstein Barr, du virus HIV, du virus de l'hépatite B (EP 185 573), du virus de la pseudo-rage, du "syncitia forming virus", d'autres virus ou encore de peptides antigéniques spécifiques de tumeurs (EP 259 212).

Préférentiellement, l'acide nucléique comprend également des séquences permettant l'expression du gène d'intérêt thérapeutique et/ou du gène codant pour le peptide antigénique dans la cellule ou l'organe désiré. Il peut s'agir des séquences qui sont naturellement responsables de l'expression du gène considéré lorsque ces séquences sont

susceptibles de fonctionner dans la cellule infectée. Il peut également s'agir de séquences d'origine différente (responsables de l'expression d'autres protéines, ou même synthétiques). Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus. A cet égard, on peut citer par exemple les promoteurs des gènes E1A, MLP, CMV, RSV, etc... En outre, ces séquences d'expression peuvent être modifiées par addition de séquences d'activation, de régulation, etc... il peut aussi s'agir de promoteur, inductible ou répressible.

Par ailleurs, l'acide nucléique peut également comporter, en particulier en amont du gène d'intérêt thérapeutique, une séquence signal dirigeant le produit thérapeutique synthétisé dans les voies de sécrétion de la cellule cible. Cette séquence signal peut être la séquence signal naturelle du produit thérapeutique, mais il peut également s'agir de toute autre séquence signal fonctionnelle, ou d'une séquence signal artificielle. L'acide nucléique peut également comporter une séquence signal dirigeant le produit thérapeutique synthétisé vers un compartiment particulier de la cellule.

Selon un aspect préféré de l'invention, les composés acidosensibles sont plus spécifiquement choisis parmi les composés de formule générale :

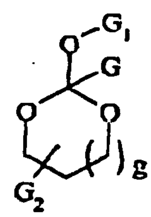

(I)

dans laquelle :

- g est un entier égal à 0 ou 1
- G représente un substituant choisi parmi l'atome d'hydrogène, les substituants alkyles comprenant 1 à 6 atomes de carbone, saturé ou insaturé, en chaîne droite ou ramifiée, ou les aryles contenant 6 à 14 atomes de carbone, et
- $G_1$ et $G_2$ représentent :

  (a) l'un un substituant hydrophile choisi parmi les radicaux de type polyamine, et l'autre un substituant hydrophobe choisi parmi les alkyles mono- ou bicaténaires ou les dérivés de stéroïde, ou bien

  (b) l'un un groupe alkyle linéaire hydrophobe comprenant 10 à 24 atomes de carbones et comprenant éventuellement une ou plusieurs insaturations, et l'autre un groupe de formule générale :

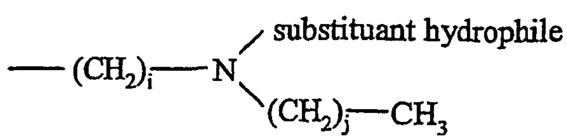

(II)

  dans laquelle i est un entier allant de 1 à 4 et j est un entier allant de 9 à 23, et le substituant hydrophile est choisi parmi les radicaux de type polyamine, ou bien

  (c) l'un un substituant hydrophile choisi parmi les polyalkylène glycols et l'autre un substituant choisi parmi les polyalkylène imines, ou bien

  (d) l'un un substituant hydrophile choisi parmi les polyalkylène glycols et l'autre un substituant hydrophobe choisi parmi les alkyles mono- ou bicaténaires, les dérivés de stéroïde, ou les conjugués covalents entre un alkyle mono- ou bicaténaire ou un dérivé de stéroïde, et une molécule de polyalkylène glycol comprenant 1 à 20 unités monomériques, ou bien

  (e) l'un un substituant hydrophile choisi parmi les polyalkylène glycols et l'autre une molécule thérapeutique.

Encore plus préférentiellement, les composés acidosensibles de l'invention sont choisis parmi les composés de formule générale :

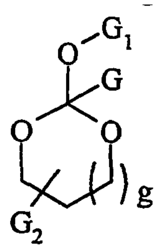

(I)

dans laquelle :

- g est un entier égal à 0 ou 1,
- G représente un substituant choisi parmi l'atome d'hydrogène, les radicaux alkyles comprenant 1 à 6 atomes de carbone, saturé ou insaturé, en chaîne droite ou ramifiée, ou le phényle, et
- $G_1$ et $G_2$ représentent :

(a) l'un un substituant hydrophile choisi parmi les radicaux de type polyamine, et l'autre un substituant hydrophobe choisi parmi les alkyles mono- ou bicaténaires ou les dérivés de stéroïde, ou bien
(d) l'un un substituant hydrophile choisi parmi les polyalkylène glycols et l'autre un substituant hydrophobe choisi parmi les alkyles mono- ou bicaténaires ou les dérivés de stéroïde.

Les nouveaux composés acidosensibles de formule générale (I) peuvent se présenter sous forme de sels non toxiques et pharmaceutiquement acceptables. Ces sels non toxiques comprennent les sels avec les acides minéraux (acides chlorhydrique, sulfurique, bromhydrique, phosphorique, nitrique) ou avec les acides organiques (acides acétique, propionique, succinique, maléique, hydroxymaléique, benzoïque, fumarique, méthanesulfonique ou oxalique).

Les composés acidosensibles selon la présente invention peuvent être préparés selon de nombreuses méthodes choisies parmi celles décrites dans la littérature pour la synthèse de molécules contenant un groupe orthoester cyclique (par exemple, on peut se référer aux exemples donnés dans la revue Synthesis, Robert H. DeWolfe, 1974, pp. 153-172). Selon une alternative envisageable, les composés acidosensibles de formule générale (I) peuvent par exemple être obtenus par réaction d'un alcool de formule $G_1OH$ sur un orthoester de formule générale :

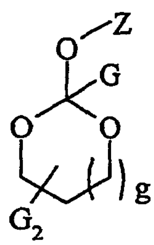

(III)

dans lesquelles g, G, $G_1$ et $G_2$ sont tels que définis pour la formule générale (I), et Z représente un groupe alkyle linéaire ou ramifié contenant 1 à 4 atomes de carbone.

Cette substitution peut être effectuée en présence d'un catalyseur acide et/ou peut être activée thermiquement à température comprise entre 50 et 150°C, avec ou sans solvant Si on choisit d'opérer en présence d'un solvant, ce dernier est choisi parmi les solvants classiques de la chimie organique comme par exemple les solvants organochlorés, les solvants aromatiques ou encore les éthers. Lorsqu'un catalyseur est utilisé, il peut s'agir d'un acide minéral ou organique, d'un acide de Lewis ou de Brönsted. Par exemple, le catalyseur peut être choisi parmi l'acide chlorhydrique, l'acide sulfurique, l'acide paratoluènesulfonique, l'acide camphorsulfonique, le paratoluènesulfonate de pyridinium, ou encore le chlorure de magnésium. Cette substitution peut aussi être favorisée en distillant l'alcool ZOH produit au cours de la réaction s'il est plus volatil que l'alcool $G_1OH$. Cette distillation en continue peut être réalisée en chauffant à pression atmosphérique ou sous pression réduite.

L'alcool de départ $G_1OH$ est soit disponible commercialement, soit il peut être synthétisé par toute méthode connue de l'homme du métier, par exemple par hydratation de l'alcène correspondant, par hydrolyse du dérivé halogéné correspondant, ou encore par réduction du dérivé carbonylé correspondant.

Selon une autre variante de l'invention, le groupe Z peut déjà représenter le groupe $G_1$ et dans ce cas, l'étape

de réaction entre l'orthoester de formule générale (III) et l'alcool $G_1OH$ n'est pas nécessaire.

Le composé de formule générale (III) peut être obtenu par action d'un trialkylorthoester de formule générale (IV) :

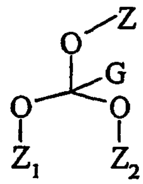

(IV)

dans laquelle Z et G sont tels que définis précédemment, et $Z_1$ et $Z_2$, identiques ou différents, représentent des groupes alkyles linéaires ou ramifiés contenant 1 à 4 atomes de carbone,
sur un diol de formule générale (V) :

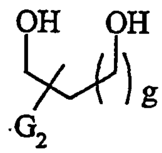

(V)

dans laquelle g et $G_2$ sont tels que définis précédemment.

La réaction peut être effectuée selon les méthodes classiques de protection des diols en orthoester, par exemple selon les méthodes indiquées par T. W. Greene et P.G.M. Wuts dans *« Protective Groups in Organic Synthesis »* (2$^{nd}$ Ed., Wiley-Interscience, pp. 135-136). On opère généralement dans un solvant organique classique (par exemple les solvants organo-chlorés, aromatiques, les éther, ...) en présence d'un catalyseur acide. Le catalyseur peut être choisi parmi les acides minéraux ou organiques, de Lewis ou de Brönsted. Par exemple, on peut utiliser l'acide chlorhydrique, l'acide sulfurique, l'acide paratoluènesulfonique, l'acide camphorsulfonique, le paratoluènesulfonate de pyridinium, ou encore le chlorure de magnésium.

Le trialkylorthoester de formule générale (IV) est soit disponible commercialement, soit il peut être synthétisé selon les méthodes classiques connues de l'homme de l'art, par exemple à partir de l'ester correspondant, ou encore par substitution des groupes alkoxy à partir d'un autre trialkylorthoester commercial.

Le diol de formule générale (V) est soit disponible commercialement, soit il peut être obtenu par réaction entre un diol commercial et $G_2$, ou bien encore il peut être obtenu par fonctionnalisation directe de $G_2$ en diol. Cette fonctionnalisation peut par exemple consister en une oxydation de l'alcène correspondant, ou bien èn l'ouverture d'un époxyde correspondant, selon les méthodologies bien connues de l'homme de l'art.

Lorsque l'un de $G_1$ ou de $G_2$ représente un radical de type polyamine, celui-ci est soit commercial, soit il est obtenu selon les méthodes classiques connues de l'homme du métier, par exemple selon les méthodes décrites dans l'art antérieur (par exemple dans les publications WO 96/17823, WO 97/18185, WO 98/54130 ou encore WO 99/51581), ou selon des méthodes analogues.

Lorsque l'un de $G_1$ ou de $G_2$ représente un substituant hydrophobe choisi parmi les alkyles mono- ou bicaténaires, celui-ci est soit commercial, soit il est obtenu selon les méthodes classiques connues de l'homme du métier. Par exemple, lorsqu'il s'agit d'un substituant dialkylamino à longue chaîne carbonées, celui-ci peut être préparé à partir de l'amine primaire correspondante par alkylation (monosubstitution d'un alkyle halogéné), par réduction alkylative (à partir d'un aldéhyde), ou encore par condensation/réduction (formation d'une fonction amide à partir d'un acide puis réduction).

Lorsque l'un de $G_1$ ou de $G_2$ représente un substituant hydrophobe choisi parmi les dérivés de stéroïde ou les dendrimères hydrophobes, celui-ci est de préférence choisi parmi les produits disponibles commercialement.

Lorsque l'un de $G_1$ ou de $G_2$ représente un substituant choisi parmi les polyalkylène glycols ou les mono- ou polysaccharides, celui-ci est soit commercial, soit il est obtenu par les méthodes classiques connues de l'homme du métier, notamment par polymérisation. Dans le cas ou ce substituant est lié covalemment à un élément de ciblage, la synthèse des composés acidosensibles selon la présente invention décrite ci-avant pourra être réalisée avant ou après la fixation par les méthodes classiques de l'homme de l'art dudit élément de ciblage sur ce substituant.

Lorsque l'un de $G_1$ ou de $G_2$ représente un substituant choisi parmi les polyalkylène imines, celui-ci est soit commercial, soit il est obtenu selon les méthodes classiques connues de l'homme du métier ou selon les méthodes

décrites dans l'art antérieur, par exemple dans la publication WO 96/02655.

Le procédé de préparation indiqué ci-avant ne constitue qu'un procédé donné à titre illustratif, et tout autre procédé de préparation équivalent peut naturellement être également mis en oeuvre. Par exemple, il est possible d'effectuer les réactions à partir d'un diol de formule générale (V) ne possédant pas le groupe $G_2$ mais à la place un groupe fonctionnel éventuellement protégé (par exemple une amine protégée), en effectuant une étape supplémentaire finale de fixation du groupe $G_2$ (par exemple, déprotection de l'amine puis condensation d'un acide de formule $G_2COOH$).

Un autre objet de l'invention concerne les compositions comprenant au moins un composé acidosensible de formule générale (I) tel que défini précédemment. Selon une variante de l'invention, lesdites compositions comprennent au moins une substance biologiquement active et un composé acidosensible de formule générale (I) dans laquelle $G_1$ et $G_2$ ont les définitions indiquées sous (a), (b), (c) ou (d).

Les compositions selon l'invention peuvent en outre comporter un ou plusieurs adjuvants capables de s'associer aux complexes formés entre le composé acidosensible selon l'invention et la substance biologiquement active. Dans un autre mode de mise en oeuvre, la présente invention concerne donc les compositions comprenant au moins une substance biologiquement active, un composé acidosensible de formule (I) dans laquelle $G_1$ et $G_2$ ont les définitions indiquées sous (a), (b), (c) ou (d), et un ou plusieurs adjuvants. La présence de ce type d'adjuvants (lipides, peptides ou protéines par exemple) peut permettre avantageusement d'augmenter le pouvoir transfectant des composés dans les cas où la substance biologiquement active est un acide nucléique à transfecter.

Dans cette optique, les compositions selon la présente invention peuvent comprendre comme adjuvant, un ou plusieurs lipides neutres. Il a en effet été montré que l'addition d'un lipide neutre permet d'améliorer la formation des particules nucléolipidiques (dans le cas où la substance biologiquement active est un acide nucléique), et de favoriser la pénétration de la particule dans la cellule en déstabilisant sa membrane.

Plus préférentiellement, lesdits lipides neutres sont des lipides à deux chaînes grasses. De manière particulièrement avantageuse, on utilise des lipides naturels ou synthétiques, zwitterioniques ou dépourvus de charge ionique dans les conditions physiologiques. Il peuvent être choisis plus particulièrement parmi la dioléoylphosphatidyléthanolamine (DOPE), l'oléoylpalmitoylphosphatidyléthanolamine (POPE), le distéaroyl phosphatidyl-éthanolamine, le dipalmitoyl phosphatidyléthanolamine, le dimirystoyl phosphatidyl-éthanolamine ainsi que leurs dérivé N-méthylés 1 à 3 fois, les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

Ces différents lipides peuvent être obtenus soit par synthèse, soit par extraction à partir d'organes (exemple : le cerveau) ou d'oeufs, par des techniques classiques bien connues de l'homme du métier. En particulier, l'extraction des lipides naturels peut être réalisée au moyen de solvants organiques (voir également Lehninger, Biochemistry).

Préférentiellement, dans le cas où la substance biologiquement active est un acide nucléique, les compositions de l'invention comprennent de 0,01 à 20 équivalents d'adjuvant(s) pour un équivalent d'acides nucléiques en mol/mol et, plus préférentiellement, de 0,5 à 5.

Les composés acidosensibles selon l'invention peuvent avoir diverses utilisations selon les substituants $G_1$ et $G_2$ situés de part et d'autre de l'orthoester cyclique.

Dans le cas où les substituants $G_1$ et $G_2$ ont les définitions indiquées en (a), (b) ou (c) dans la formule générale (I), les composés acidosensibles selon l'invention peuvent former des conjugués (par exemple du type liposomes, complexes ou encore nanoparticules) directement avec des substances biologiquement actives qui peuvent ensuite être libérées dans les tissus ou compartiments cellulaires, qui sont plus acides que la normale physiologique. Ces composés acidosensibles sont notamment plus particulièrement utiles pour la transfection d'acides nucléiques.

Dans le cas où les substituants $G_1$ et $G_2$ ont les définitions indiquées en (d) dans la formule générale (I), les composés acidosensibles selon l'invention constituent des agents de surface non-ioniques permettant à la fois de stabiliser des particules encapsulant une substance biologiquement active et de libérer ladite substance biologiquement active par dégradation dans les régions très faiblement acides à acides de l'organisme, en particulier des régions où le pH est acide et est compris entre environ 4 et environ 7.

En outre, les substituants polysaccharide ou polyalkylène glycol, et plus spécifiquement le polyéthylène glycol (PEG), sont connues pour conférer une sorte de « furtivité » aux particules auxquelles elles sont associées en inhibant l'adsorption non-spécifique par les protéines du sérum, et par conséquent la reconnaissance des dites particules par les macrophages (voir par exemple Torchilin et al., Biochim. Biophys. Acta 1994, 1195, pp. 11-20 ou Papahadjopoulos et al., PNAS 1991, 88, p. 11460-4). Ainsi, les composés acidosensibles comportant une molécule de PEG selon l'invention présentent un avantage sur le plan de l'innocuité et également un avantage supplémentaire en ce sens qu'elles diminuent le risque d'interférences avec d'autres protéines. Au niveau des régions acides de l'organisme, la dégradation de l'orthoester présent dans les composés selon l'invention permet la séparation des molécules de PEG du reste de la particule, rendant la substance biologiquement active à nouveau « disponibles » (il y a en fait « disparition de la furtivité »). On peut ainsi espérer un transfert sélectif vis à vis des tissus acides.

Enfin, dans le cas où les substituants $G_1$ et $G_2$ ont les définitions indiquées en (e) ou (f) dans la formule générale (I), les composés acidosensibles selon l'invention constituent des conjugués covalents avec une molécule thérapeutique permettant ainsi sa vectorisation puis sa libération dans les régions acides de l'organisme. Ces conjugués covalents sont de même nature que ceux décrits par Kratz et al., mais avec une nouvelle liaison acidosensible entre la molécule thérapeutique et la partie « vecteur », qui présente l'avantage d'avoir une sensibilité modulable par rapport aux liaisons pH-sensibles utilisées jusque là.

Ainsi, la présente invention a également pour objet l'utilisation des composés acidosensibles de formule générale (I) tels que définis ci-avant pour fabriquer un médicament destiné à soigner les maladies. Dans ce cas, la maladie visée conditionne le choix de la substance biologiquement active.

Selon une variante particulièrement avantageuse, lorsque la substance biologiquement active est un acide nucléique, les composés acidosensibles de formule générale (I) dans la quelle $G_1$ e $G_2$ ont les définitions indiquées sous (a), (b) ou (c) peuvent être utilisés pour fabriquer un médicament destiné à la transfection *in vitro, ex vivo* ou *in vivo* d'acides nucléiques, en particulier dans les cellules primaires ou dans les lignées établies. Il peut s'agir par exemple de cellules fibroblastiques, musculaires, nerveuses (neurones, astrocytes, cellules glyales), hépatiques, de la lignée hématopoiétique (lymphocytes, CD34, dendritiques...), ou encore épithéliales, sous forme différenciées ou pluripotentes (précurseurs).

Enfin, selon une autre alternative de l'invention, les composés acidosensibles de formule générale (I) dans laquelle $G_1$ e $G_2$ ont les définitions indiquées sous (e) ou (f) peuvent être utilisés comme médicament.

Dans tout ce qui précède, les composés acidosensibles selon la présente invention se dégradent dans les tissus ou compartiments cellulaires dont le pH est plus acide que la normale physiologique. Cependant, selon une autre alternative, il est possible d'induire ou augmenter l'acidité dans la zone cible de l'organisme par un traitement général ou local connu de l'homme de l'art. On peut citer à titre d'exemple, de manière non limitante, l'injection d'un produit acide dans la zone à traiter ou encore l'injection intraveineuse de glucose qui entraîne une acidification spécifique des tissus tumoraux (T. Volk et al. ; Br. J. Cancer ; 1993, 68 (3), 492-500). Ainsi, les composés acidosensibles selon la présente invention peuvent également être utilisés dans des zones de l'organisme a priori non-acides et qui ont été rendues acides par des traitements connus de l'homme de l'art.

Pour toute les utilisations des composés acidosensibles selon la présente invention indiquées ci-avant, les compositions selon l'invention comprenant :

- soit un composé acidosensible de formule générale (I) dans laquelle $G_1$ et $G_2$ ont les définitions indiquées sous (e) ou (f),
- soit un composé acidosensible de formule générale (I) dans laquelle $G_1$ et $G_2$ ont les définitions indiquées sous (a), (b), (c) ou (d) et une substance biologiquement active,

peuvent être formulées en vue d'administrations par exemple par voie topique, cutanée, orale, rectale, vaginale, parentérale, intranasale, intraveineuse, intramusculaire, sous-cutanée, intraoculaire, transdermique, intratrachéale, ou encore intrapéritonéale. De préférence, les compositions de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses de substances biologiquement actives utilisées pour l'injection ainsi que le nombre d'administrations peuvent être adaptées en fonction de différents paramètres, et notamment en fonction du mode d'administration utilisé, de la pathologie concernée, du gène à exprimer lorsque la substance biologiquement active est un acide nucléique, ou encore de la durée du traitement recherchée. En ce qui concerne plus particulièrement le mode d'administration, il peut s'agir soit d'une injection directe dans les tissus, par exemple au niveau des tumeurs, ou les voies circulatoires, soit d'un traitement de cellules en culture suivi de leur réimplantation *in vivo*, par injection ou greffe. Les tissus concernés dans le cadre de la présente invention sont par exemple les muscles, la peau, le cerveau, les poumons, le foie, la rate, la moelle osseuse, le thymus, le coeur, la lymphe, le sang, les os, les cartilages, le pancréas, les reins, la vessie, l'estomac, les intestins, les testicules, les ovaires, le rectum, le système nerveux, les yeux, les glandes, ou encore les tissus conjonctifs.

Outre les dispositions qui précèdent, la présente invention comprend également d'autres caractéristiques et avantages qui ressortiront des exemples et figures qui suivent, et qui doivent être considérés comme illustrant l'invention sans en limiter la portée. Notamment, la Demanderesse propose à titre non-limitatif divers protocoles opératoires ainsi que des intermédiaires réactionnels susceptibles d'être mis en oeuvre pour préparer les composés de formule générale (I). Bien entendu, il est à la portée de l'homme du métier de s'inspirer de ces protocoles et/ou produits intermédiaires pour mettre au point des procédures analogues en vue de conduire à d'autres composés de formule générale (I) selon l'invention.

## FIGURES

Figure 1 : Evolution du taux de fluorescence en fonction du temps à pH 5 de complexes formés entre l'ADN et un lipide cationique témoin ou bien les composés acidosensibles A Syn ou Trans, à 3 rapports différents : 0,4 ou 1,7 ou 6,0 nmol de lipide cationique ou de composé acidosensible/µg d'ADN.

Figure 2: Efficacité de transfection *in vitro* dans des cellules HeLa de complexes formés entre l'ADN et le composé A Syn ou Trans ou un lipide cationique témoin, à diférents rapports de charges, avec ou sans sérum.
L'axe des ordonnées représente l'expression de la luciférase en pg/puit/µg de protéine. L'axe des absisses indique le rapport de charges composé A Syn ou Trans ou lipide cationique témoin/ADN.

Figure 3 : Evolution de la taille (en nm) de particules nucléolipidiques lipide cationique témoin/ADN en fonction de la quantité de composé C ou composé D ou de Brij 700 ou d'analogue non-acidosensible du composé D (Analogue D) utilisée par rapport à la quantité d'ADN (poids/poids). Une faible taille indique que les particules nucléolipidiques sont stabilisées. Une taille très élevée indique au contraire une déstabilisation des particules nucléolipidiques qui ont alors tendance à s'agréger.

Figure 4: Evolution de la taille (en nm) de particules nucléolipidiques lipide cationique témoin/ADN/composé ou analogue D en fonction du temps, à différents rapports (poids/poids), lorsque le pH est de 5. Une faible taille des particules nucléolipidiques indique que celles-ci sont stabilisées. Une taille très élevée indique au contraire une déstabilisation des particules nucléolipidiques qui ont alors tendance à s'agréger.

Figure 5 : Evolution de la taille (en nm) de particules nucléolipidiques lipide cationique témoin/ADN/composé C ou composé E en fonction du temps, à différents pH (pH 4, pH 5, pH 6 et pH 7,4). Le rapport composé C ou composé E/ADN est fixé à 1 (en nmoles/µg d'ADN).

Figure 6 : Représentation schématique du plasmide pXL3031.

Figure 7 : Courbe dose-réponse du composé D sur l'activité de transfert de gène *in vivo* médié par les complexes lipide cationique/DOPE/ADN (5/5/1). Le composé « Analogue D » est utilisé comme contrôle négatif. Les données sont des moyennes (lignes) et des valeurs individuelles (points) de 4 souris Balb/C portant des tumeurs M109 sous-cutanées.

## EXEMPLES

Les réactifs et catalyseurs usuels tels que la triéthylamine, l'acide trifluoroacétique, l'anhydride trifluoroacétique, le bromoacétate de *tert*-butyl, la butyrolactone, le 3-aminopropan-1,2-diol, le sérinol (2-aminopropan-1,3-diol), le triméthylorthoformate, le triméthylorthoacétate, l'acide para-toluène sulfonique, le pyridinium para-toluène sulfonate, ou encore l'hexafluorophosphate de benzotriazol-1-yloxytris(diméthylamino)-phosphonium (BOP), sont disponibles commercialement.

Les lavages sont réalisés par des solutions aqueuses saturées en chlorure de sodium, saturées en hydrogénocarbonate de sodium et par une solution d'hydrogénosulfate de potassium concentrée à 0,5 mol/l.

Les polymères hydrophiles (polyéthylène glycols de différentes tailles) sont disponibles commercialement. Les substituants hydrophiles de type polyamines sont également disponibles commercialement ou bien ils peuvent être synthétisés par des méthodes classiques connues de l'homme du métier comme indiqué notamment dans les exemples qui suivent. Les substituants hydrophobes (dialkylamines mono- ou bi-caténaires, alcools gras, ...) sont commerciaux ou bien synthétisés selon les méthodes classiques connues de l'homme du métier. Par exemple, les dialkylamines mono- ou bi-caténaires peuvent être synthétisées à partir des amines primaires et des dérivés alkyle halogénés correspondants comme indiqué dans les exemples qui suivent.

Les spectres de Résonance Magnétique Nucléaire du Proton (RMN $^1$H) ont été enregistrés sur des spectromètres Bruker 300, 400 et 600 MHz. Les déplacements chimiques sont exprimés en ppm (partie par million) et les multiplicités par les abréviations usuelles.

Le plasmide utilisé est le pXL3031 décrit dans la publication Gene Therapy (1999) 6, pp. 1482-1488, qui contient le gène luc codant pour la luciférase sous contrôle du promoteur P/E CMV du cytomégalovirus. Ce plasmide est représenté à la figure 6. Sa taille est de 3671 bp. La solution de plasmide utilisée est dilué à 1,262 g/l dans de l'eau pour préparation injectable.

**EXEMPLE 1 : Synthèse du 2,2,2-trifluoro-N-(2-méthoxy-[1,3]dioxolan-4-ylméthyl)-acétamide (« Ortho 1 »)**

Le 2,2,2-trifluoro-N-(2-méthoxy-[1,3]dioxolan-4-ylméthyl)-acétamide (« Ortho 1 ») a pour formule:

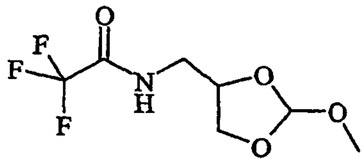

Ortho 1

Il peut être obtenu en deux étapes à partir du 3-aminopropan-1,2-diol :

1) Préparation de la N-(2,3-dihydroxy-propyl)-2,2,2-trifluoro-acétamide

Dans un ballon muni d'un barreau aimanté, 15 g de 3-aminopropan-1,2-diol (164,6 mmol) sont solubilisés dans 100 ml de tétrahydrofuranne. Le mélange réactionnel est alors refroidi à 0°C par un bain de glace et 21,5 ml de trifluoroacétate d'éthyle (181,1 mmol) sont ajoutés progressivement. Le mélange réactionnel est agité pendant 2 heures à température ambiante. Le brut réactionnel est ensuite évaporé à sec. On obtient ainsi 29 g d'une huile incolore pure (rendement : 95 %) qui est utilisée sans purification supplémentaire.

2) Préparation de la 2,2,2-trifluoro-N-(2-méthoxy-[1,3]dioxolan-4-ylméthyl)-acétamide (ortho 1)

Les 29 g de N-(2,3-dihydroxy-propyl)-2,2,2-trifluoro-acétamide obtenus à l'étape précédente (155 mmol) sont solubilisés dans 75 ml de dichlorométhane additionnés de 75 ml de triméthylorthoformate (685 mmol). 300 mg d'acide paratoluènesufonique (1,7 mmol) sont ensuite ajoutés et le mélange réactionnel est agité pendant 2 heures à température ambiante.
Ce brut est alors dilué dans 500 ml de dichlorométhane, lavé par 3 fois 200 ml d'hydrogénocarbonate de sodium saturé puis 3 fois 200 ml de chlorure de sodium saturé. La phase organique est séchée sur sulfate de magnésium, filtrée et concentrée à sec. On obtient ainsi 30 g de produit huileux pur (rendement : 85 %) sans purification supplémentaire.
R.M.N. $^1H$ (300 MHz, $CDCl_3$, $\delta$ en ppm). On observe un mélange de deux diastéréoisomères dans les proportions 50/50.

* 3,33 et 3,37 (2s : 3H en totalité) ; de 3,35 à 3,80 (mts : 3H) ; de 4,10 à 4,25 (mt : 1H) ; 4,50 (mt : 1H) ; 5,73 et 5,78 (2s : 1H en totalité) ; 6,66 et 7,55 (2 mfs : 1H en totalité).

**EXEMPLE 2 : Synthèse du 2,2,2-trifluoro-N-(2-méthoxy-2-méthyl-[1,3]dioxan-5-yl)-acétamide (« Ortho 2 )**

Le 2,2,2-trifluoro-N-(2-méthoxy-2-méthyl-[1,3]dioxan-5-yl)-acétamide (« Ortho 2) a pour formule:

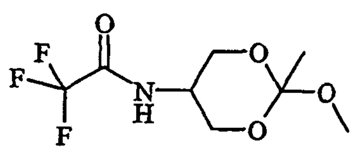

Ortho 2

Il peut être obtenu en deux étapes à partir du 2-aminopropan-1,3-diol (sérinol) :

1) Préparation de la 2,2,2-trifluoro-N-(2-hydroxy-1-hydroxyméthyl-éthyl)-acétamide

4 g de 2-aminopropan-1,3-diol (43,9 mmol) sont additionnés de 20 ml de tétrahydrofuranne. Le mélange réactionnel est alors refroidi à 0°C par un bain de glace et 5,8 ml de trifluoroacétate d'éthyle (48,3 mmol) sont ajoutés progressivement. Cette solution est agitée pendant 2 heures à température ambiante. Le mélange réactionnel est ensuite évaporé à sec, repris 3 fois dans le dichlorométhane pour évaporer totalement le tétrahydrofuranne. 8,1 g de poudre blanche (rendement : 99 %) sont obtenus purs et utilisés dans l'étape suivante sans purification supplémentaire.

2) Préparation de la 2,2,2-trifluoro-N-(2-méthoxy-2-méthyl-[1,3]dioxan-5-yl)-acétamide (Ortho 2)

7,9 g de 2,2,2-trifluoro-N-(2-hydroxy-1-hydroxyméthyl-éthyl)-acétamide obtenus à l'étape précédente (42,2 mmol) sont solubilisés dans 30 ml de dichlorométhane additionnés de 16,1 ml de triméthylorthoacétate (126,7 mmol). 73 mg d'acide paratoluènesufonique (0,42 mmol) sont ensuite ajoutés et le mélange réactionnel est agité pendant 3 heures à température ambiante.
Le brut réactionnel est ensuite dilué par 150 ml de dichlorométhane, lavé par 3 fois 50 ml d'une solution d'hydrogénocarbonate de sodium saturée, puis 3 fois 50 ml d'une solution de chlorure de sodium saturée. La phase organique est séchée sur sulfate de magnésium, filtrée et concentrée à sec. 9,9 g de solide blanc sont obtenus purs sans purification supplémentaire (rendement : 96 %).
R.M.N. $^1H$ (300 MHz, $CDCl_3$, $\delta$ en ppm). On observe un mélange de deux diastéréoisomères dans les proportions approximatives 75/25.

* 1,49 et 1,50 (2 s : 3H en totalité) ; 3,34 et 3,35 (2 s : 3H en totalité) ; 3,66 et 3,82 (respectivement dmt, J = 12 Hz et dd, J = 11 et 8 Hz : 2H en totalité) ; de 3,90 à 4,00 et de 4,20 à 4,35 (2 mts : 1H en totalité) ; 3,97 et 4,33 (respectivement dd, J =11 et 5 Hz et dmt, J = 12 Hz : 2H en totalité) ; 6,38 et 7,04 (2 mfs étalés : 1H en totalité).

**EXEMPLE 3 : Synthèse des composés Syn et Trans tétraacétate de 4-{4-[(2-{3-[4-(3-Amino-propylamino)-butylamino]-propylamino}-acetylamino)-methyl]-[1,3]dioxolan-2-yloxy}-N,N-dioctadecyl-butyramide**

Cet exemple décrit une voie de synthèse du composé acidosensible A sous ses deux formes diastéréoisomériques distinctes Syn et Trans de formule :

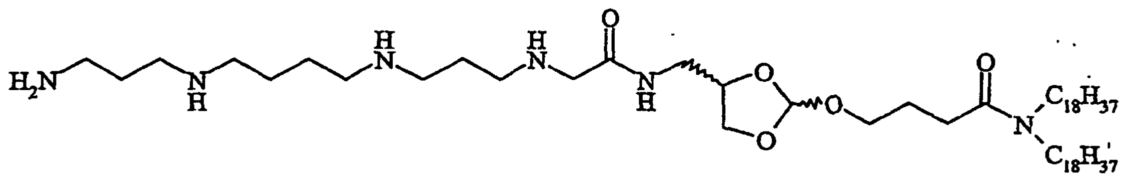

Composé A

**a - Synthèse des Syn et Trans 4-(4-Aminométhyl-[1,3]dioxolan-2-yloxy)-N,N-dioctadécyl-butyramide (partie lipide-O-Ortho 1-$NH_2$)**

Cette synthèse procède en trois étapes : fonctionnalisation de la dioctadécylamine en alcool et fixation sur le groupe Ortho 1 dont le groupe protecteur est ensuite clivé.

1) Préparation de la 4-hydroxy-N,N-dioctadécyl-butyramide

4,6 g de chlorure d'aluminium (34 mmol) sont additionnés de 25 ml de chloroforme, le tout étant refroidi à 10°C environ par un bain thermostaté. 6,4 ml de triéthylamine (46 mmol) dans 15 ml de chloroforme sont ajoutés goutte à goutte puis on laisse le mélange réactionnel revenir à température ambiante. 6 g de dioctadécylamine (11,5 mmol) mélangés à 1 ml de butyrolactone (13,8 mmol) dans 110 ml de chloroforme sont ajoutés progressivement au mélange par une ampoule de coulée.
La réaction n'évolue plus après 2 heures sous agitation magnétique à température ambiante. 75 ml d'eau sont alors ajoutés et le mélange réactionnel est agité pendant 30 minutes. Le brut est filtré sur Célite puis lavé au chloroforme. Le filtrat est décanté et la phase organique est lavée par 3 fois 50 ml d'une solution de chlorure de sodium saturée. La solution chloroformique est séchée sur sulfate de magnésium, filtrée et concentrée. 4,9 g de poudre blanche sont obtenus après chromatographie sur silice (rendement : 70 %).

2) Préparation des Syn et Trans N,N-Dioctadécyl-4-{4-[(2,2,2-trifluoro-acétylamino)-méthyl]-[1,3]dioxolan-2-yloxy}-butyramides

2,8 g de 4-hydroxy-N,N-dioctadécyl-butyramide obtenue à l'étape précédente (4,6 mmol) sont mélangés à 2,6 g de 2,2,2-trifluoro-N-(2-méthoxy-[1,3]dioxolan-4-ylméthyl)-acetamide (Ortho 1, 11,5 mmol) et 90 mg de chlorure de magnésium (0,92 mmol). Le tout est chauffé sans solvant à 80°C pendant deux heures.

Le brut réactionnel est ensuite dissout dans 150 ml de cyclohexane et lavé par 3 fois 30 ml d'une solution d'hydrogénocarbonate de sodium saturée, puis par 3 fois 30 ml d'une solution de chlorure de sodium saturée. La phase organique est séchée sur sulfate de magnésium, filtrée et concentrée. La purification est effectuée par chromatographie sur silice. On isole ainsi 1,2 g et 1,1 g des deux diastéréoisomères Syn et Trans attendus sous forme de poudre blanche (rendement : 62 %).
R.M.N. $^1H$ du composé SYN (300 MHz, $CDCl_3$, $\delta$ en ppm) : 0,89 (t, J = 7 Hz : 6H) ; de 1,15 à 1,40 (mt : 60H) ; 1,52 (mt : 4H) ; 1,94 (mt : 2H) ; 2,39 (t, J = 7 Hz : 2H) ; 3,20 (t large, J = 8 Hz : 2H) ; 3,29 (mt : 2H) ; 3,61 (t, J = 5 Hz : 2H) ; 3,66 (mt : 2H) ; 3,79 (dd, J = 8 et 7 Hz : 1H) ; 4,11 (t, J = 8 Hz : 1H) ; 4,50 (mt : 1H) ; 5,82 (s : 1H) ; 8,13 (mf : 1H).
R.M.N. $^1H$ du composé TRANS (300 MHz, $CDCl_3$, $\delta$ en ppm) : 0,89 (t, J = 7 Hz : 6H) ; de 1,15 à 1,40 (mt : 60H) ; 1,52 (mt : 4H) ; 1,95 (mt : 2H) ; 2,38 (t, J = 7 Hz : 2H) ; 3,21 (t large, J = 8 Hz : 2H) ; 3,29 (t large, J = 8 Hz : 2H) ; 3,44 (mt : 1H) ; de 3,55 à 3,75 (mt : 1H) ; 3,60 (t, J = 6 Hz : 2H) ; 3,69 (dd, J = 8,5 et 5 Hz : 1H) ; 4,19 (dd, J = 8,5 et 7 Hz : 1H) ; 4,50 (mt : 1H) ; 5,87 (s : 1H) ; 6,70 (mf : 1H).

<u>3) Préparation des Syn et Trans 4-(4-Aminométhyl-[1,3]dioxolan-2-yloxy)-N,N-dioctadécyl-butyramide</u>

1,1 g de N,N-dioctadécyl-4-{4-[(2,2,2-trifluoro-acétylamino)-méthyl]-[1,3]dioxolan-2-yloxy}-butyramide obtenus à l'étape précédente (1,37 mmol, Syn ou Trans) sont dissout dans 10 ml de tétrahydrofuranne et 10 ml de soude molaire à 4% sont additionnés sous forte agitation. La réaction est laissée une nuit à température ambiante.
Le tétrahydrofuranne est ensuite concentré puis le produit est extrait par trois fois 150 ml d'éther diéthylique. La phase organique est séchée sur chlorure de calcium, filtrée et évaporée. 840 mg des produits attendus sont ainsi isolés (rendement : 86%), et utilisés tels quels pour la suite.

**<u>b - Synthèse de l'acide (trifluoroacétyl-{3-[trifluoroacétyl-(4-{trifluoroacétyl-[3-(2,2,2-trifluoro-acétylamino)-propyl]-amino}-butyl)-amino]-propyl}-amino)-acétique (partie hydrophile polyamine-COOH)</u>**

Cette synthèse procède en deux étapes : protection des quatre amines de la spermine puis substitution d'une des amines primaires par l'acide bromoacétique protégé.

<u>1) Synthèse de la 2,2,2-trifluoro-N-[3-(2,2,2-trifluoro-acétylamino)-propyl]-N-(4-{trifluoroacétyl-[3-(2,2,2-trifluoro-acétylamino)-propyl]-amino}-butyl)-acétamide</u>

8 g de spermine (39,5 mmol) sont solubilisés dans 75 ml de dichlorométhane. 33 ml de triéthylamine (237 mmol) sont ajoutés puis le mélange réactionnel est refroidi à 0°C par un bain de glace. 41,5 g d'anhydride trifluoroacétique dilués dans 100 ml de dichlorométhane sont alors additionnés goutte à goutte pendant 1 heure par une ampoule de coulée. On laisse ensuite le mélange réactionnel revenir à température ambiante et la réaction est laissée une nuit sous agitation.
75 ml d'une solution à 5% d'hydrogénocarbonate de sodium sont ensuite ajoutés au mélange réactionnel et la solution est agitée 15 minutes à température ambiante. La phase aqueuse est extraite par 3 fois 150 ml de dichlorométhane. Les phases organiques sont rassemblées et lavées par 3 fois 100 ml d'une solution d'hydrogénosulfate de potassium concentrée à 0,5 M, puis 3 fois 100 ml d'une solution de chlorure de sodium saturée. La phase organique est ensuite séchée sur sulfate de magnésium, filtrée et concentrée à sec. 22,5 g d'une poudre jaune purs sont isolés sans purification supplémentaire (rendement : 97 %).

<u>2) Préparation de l'acide (trifluoroacétyl-{3-[trifluoroacétyl-(4-{trifluoroacétyl-[3-(2,2,2-trifluoro-acétylamino)-propyl]-amino}-butyl)-amino]-propyl}-amino)-acétique</u>

1 g d'hydrure de sodium (60 % dans l'huile soit 25,6 mmol) sont additionnés de 60 ml de diméthylformamide sèche. Le mélange réactionnel sous flux d'argon est refroidi par un bain de glace puis 10 g de la 2,2,2-trifluoro-N-[3-(2,2,2-trifluoroacétylamino)-propyl]-N-(4-{trifluoroacétyl-[3-(2,2,2-trifluoro-acétylamino)-propyl]-amino}-butyl)-acetamide obtenus précédemment (17 mmol) solubilisés dans 40 ml de diméthylformamide sèche sont additionnés goutte à goutte. Le mélange réactionnel est laissé 1 heure à température ambiante puis est de nouveau refroidi par un bain de glace et 3,66 g de bromoacétate de *tert*-butyle (18,7 mmol) sont ajoutés. Le mélange réactionnel est agité pendant une nuit à température ambiante.
500 ml d'acétate d'éthyle sont ensuite ajoutés puis le mélange est lavé par trois fois 100 ml d'une solution d'hydrogénocarbonate de sodium saturée, et trois fois 100 ml d'une solution de chlorure de sodium saturée. La phase organique est ensuite séchée sur sulfate de magnésium, filtrée et concentrée à sec. On isole ainsi une huile jaune contenant le produit attendu impur, sous forme de *tert*-butyl ester.
Ce brut réactionnel est dilué dans 50 ml de dichlorométhane et 50 ml d'acide trifluoroacétique sont additionnés. La

solution est agitée pendant 3 heures à température ambiante. Le mélange réactionnel est ensuite évaporé à sec puis dilué dans 50 ml de dichlorométhane. Le produit est alors extrait par 3 fois 150 ml d'une solution d'hydrogénocarbonate de sodium saturée. La phase aqueuse obtenue est lavée par 3 fois 30 ml de dichlorométhane puis est acidifiée par ajout d'acide chlorhydrique concentré. Le produit est alors extrait par 3 fois 300 ml de dichlorométhane. La phase organique est séchée sur sulfate de magnésium, filtrée et concentrée à sec. La purification est poursuivie par chromatographie sur silice (élution : dichlorométhane/méthanol 8/2). 3,5 g d'une poudre jaune sont ainsi recueillis (rendement total sur les deux étapes : 32 %).
R.M.N. $^1H$ (400 MHz, $(CD_3)_2SO$ d6 à une température de 373K, δ en ppm) : 1,62 (mt : 4H) ; de 1,80 à 2,00 (mt : 4H) ; 3,28 (mt : 2H) ; de 3,30 à 3,60 (mt : 10H) ; 4,01 (s : 2H) ; de 9,15 à 9,35 (mf : 1H).

**c - Synthèse des Syn et Trans tétraacétate de 4-{4-[(2-{3-[4-(3-Aminopropylamino)-butylamino]-propylamino}-acétylamino)-méthyl]-[1,3]dioxolan-2-yloxy}-N,N-dioctadécyl-butyramides (composé acidosensible A**

Cette synthèse procède en trois étapes : condensation des deux molécules dont la synthèse vient d'être décrite en a et b, puis déprotection de la polyamine et enfin salification. Le même protocole a été utilisé pour les produits Syn et Trans.

1) Synthèse des Syn et Trans N,N-Dioctadécyl-4-(4-{[2-(trifluoroacétyl-{3-[trifluoroacétyl-(4-{trifluoroacétyl-[3-(2,2,2-trifluoro-acétylamino)-propyl]-amino} butyl)-amino]-propyl}-amino)-acétylamino]-méthyl}-[1,3]dioxolan-2-yloxy)-butyramides

800 mg de 4-(4-Aminométhyl-[1,3]dioxolan-2-yloxy)-N,N-dioctadécyl-butyramide (1,13 mmol Syn ou Trans) obtenus précédemment (étape a) dissout dans 10 ml de dichlorométhane sont additionnés successivement de 390 µl de triéthylamine (2,8 mmol), de 800 mg d'acide (trifluoroacétyl-{3-[trifluoroacétyl-(4-{trifluoroacétyl-[3-(2,2,2-trifluoro-acétylamino)-propyl]-amino}-butyl)-amino]-propyl}-amino)-acétique (1,24 mmol) obtenus précédemment à l'étape b et de 600 mg de BOP. La solution est agitée 2 heures à température ambiante.
Le brut réactionnel est ensuite concentré, repris dans 150 ml d'acétate d'éthyle, lavé par trois fois 40 ml d'une solution d'hydrogénocarbonate de sodium saturée, puis trois fois 40 ml d'une solution de chlorure de sodium saturée. Après séchage sur sulfate de magnésium, filtration et évaporation, le produit est purifié par chromatographie sur silice (élution : acétate d'éthyle). 1,1 g de poudre blanche sont ainsi isolés (rendement : 73 %).

2) Préparation des Syn et Trans 4-{4-[(2-{3-[4-(3-amino-propylamino)-butylamino]-propylamino}-acétylamino)-méthyl] -[1,3]dioxolan-2-yloxy}-N,N-dioctadécyl-butyramides

290 mg de N,N- dioctadécyl-4-(4-{[2- (trifluoroacétyl- {3- [trifluoroacétyl- (4-{trifluoroacétyl- [3-(2,2,2-trifluoro-acétylamino)- propyl]- amino}- butyl)- amino]-propyl}-amino)-acétylamino]- méthyl}- [1,3]dioxolan-2-yloxy)- butyramide (0,22 mmol, Syn ou Trans) obtenus précédemment sont dissout dans 3 ml de tétrahydrofuranne, et 3 ml de soude molaire à 4 % sont additionnés sous forte agitation. La réaction est laissée une nuit à température ambiante.
Le solvant est ensuite concentré puis le brut est repris dans un mélange dichlorométhane/méthanol 1/1. Cette solution brute est purifiée par chromatographie sur silice (dichlorométhane/méthanol/ammoniac, 45/45/10). Le produit est concentré puis lyophilisé après adjonction d'eau. 180 mg de lyophilisât blanc sont ainsi obtenus (rendement : 87 %).

3) Préparation des diastéréoisomères Syn et Trans du tétraacétate de 4-{4-[(2-{3-[4-(3-Amino-propylamino)-butylamino]-propylamino}-acétylamino)-méthyl]-[1,3]dioxolan-2-yloxy}-N,N-dioctadécyl-butyramide (composé A)

Le produit obtenu à l'étape précédente sous forme de base libre est ensuite salifié quantitativement sur résine échangeuse d'ions : il est solubilisé dans un mélange eau/éthanol et est élué dans une colonne contenant un large excès de résine acétate (BIO-RAD ; AG 1-X2 Resin).
R.M.N. $^1H$ du composé SYN (400 MHz, $CDCl_3$, δ en ppm) : 0,89 (t, J = 7 Hz : 6H) ; de 1,20 à 1,40 (mt : 60H) ; 1,52 (mt : 4H) ; de 1,65 à 1,90 (mt : 8H) ; 1,93 (mt : 2H) ; 1,97 (s : 3H) ; 2,37 (t, J = 7 Hz : 2H) ; 2,73 (mt : 4H) ; 2,81 (t, J = 6,5 Hz : 2H) ; 2,89 (mt : 4H) ; 2,95 (t, J = 6,5 Hz : 2H) ; de 3,15 à 3,30 (mt : 4H) ; 3,32 (AB, J = 17 Hz : 2H) ; 3,45 (dt, J = 14 et 6,5 Hz : 1H) ; de 3,55 à 3,65 (mt : 1H) ; 3,61 (t dédoublé, J = 7 et 2 Hz : 2H) ; 3,74 (t, J = 8 Hz : 1H) ; 4,06 (t, J = 8 Hz : 1H) ; 4,31 (mt : 1H) ; 5,79 (s : 1H) ; 7,81 (t, J = 5,5 Hz : 1H).
R.M.N. $^1H$ du composé TRANS (400 MHz, $CDCl_3$, δ en ppm) : 0,88 (t, J = 7 Hz : 6H) ; de 1,05 à 1,45 (mt : 60H) ; 1,51 (mt : 4H) ; de 1,65 à 1,90 (mt : 8H) ; 1,92 (mt : 2H) ; 1,97 (s : 3H) ; 2,37 (t, J = 7 Hz : 2H) ; 2,73 (mt : 4H) ; 2,80 (t, J = 6 Hz : 2H) ; 2,88 (mt : 4H) ; 2,96 (t, J = 6 Hz : 2H) ; de 3,15 à 3,55 (mt : 8H) ; 3,57 (t large, J = 6 Hz : 2H) ; 3,69 (dd, J = 7,5 et 5,5 Hz : 1H) ; 4,11 (t, J = 7,5 Hz : 1H) ; 4,43 (mt : 1H) ; 5,85 (s : 1H) ; 7,73 (t large, J = 5,5 Hz : 1H).

**EXEMPLE 4: Syntèse du tétrachlorhydrate de 4-{4-[(2-{3-[Bis-(3-aminopropyl)-amino]-propylamino}-acétylamino)-méthyl]-[1,3]dioxolan-2-yloxy}-N,N-ditétradécyl-butyramide**

Cet exemple décrit une voie de synthèse du composé acidosensible B, sous la forme d'un mélange équimolaire des deux diastéréoisomères Syn et Trans, de formule :

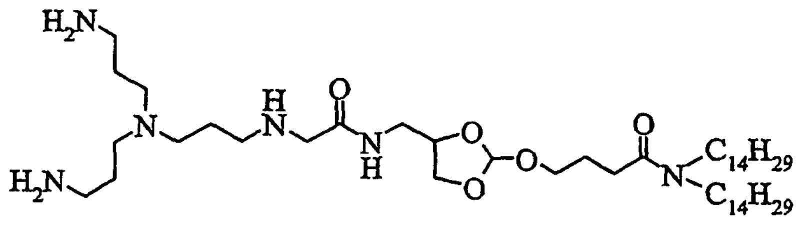

Composé B

**a - Synthèse du 4-(4-aminométhyl-[1,3]dioxolan-2-yloxy)-N,N-ditétradécyl-butyramide (partie lipide-O-Ortho 1-$NH_2$)**

Cette synthèse procède en quatre étapes : synthèse de la ditétradécylamine qui est ensuite fonctionnalisée en alcool puis fixée sur le groupe Ortho 1 dont le groupe protecteur est ensuite clivé.

1) Chlorhydrate de ditétradécylamine

74 g de bromotétradécane (267,1 mmol) sont additionnés de 400 ml d'éthanol et 57 g de tétradécylamine (267,1 mmol). 70,8 g de carbonate de sodium (667 mmol) sont alors mis en suspension et le mélange réactionnel est chauffé au reflux pendant une nuit. Le mélange réactionnel est ensuite évaporé à sec, repris dans 1,5 1 de dichlorométhane et lavé par 3 fois 200 ml d'eau puis 1 fois 400 ml d'une solution de chlorure de sodium saturée. La phase organique est séchée sur chlorure de calcium et concentrée.
La salification est effectuée par solubilisation à chaud du brut dans 600 ml d'isopropanol additionné de 300 ml d'acide chlorhydrique 5 N dans l'isopropanol. On laisse refroidir la solution limpide ainsi obtenue ce qui induit une cristallisation du produit attendu. 48,4 g de poudre blanche floconneuse sont obtenus après filtration et lavage à l'isopropanol (rendement : 41 %).

2) Préparation de la 4-hydroxy-N,N-ditétradécyl-butyramide

22,4 g de chlorure d'aluminium (168,1 mmol) sont additionnés de 75 ml de chloroforme, le tout étant refroidi à 10°C environ par un bain d'eau glacée. 39 ml de triéthylamine (280,1 mmol) dans 100 ml de chloroforme sont ajoutés goutte à goutte puis on laisse le mélange réactionnel revenir à température ambiante. 25 g de chlorhydrate de ditétradécylamine (56 mmol) mélangés à 5,2 ml de butyrolactone (67,2 mmol) dans 350 ml de chloroforme sont ajoutés progressivement au mélange sous agitation magnétique. La réaction n'évolue plus après 2 heures à température ambiante.
200 ml d'eau sont ensuite ajoutés et le mélange réactionnel est agité pendant 30 minutes. Le brut est filtré sur Célite puis lavé au chloroforme. Le filtrat est décanté et la phase organique est lavée par trois fois 150 ml d'une solution de chlorure de sodium saturée. La solution chloroformique est séchée sur sulfate de magnésium, filtrée et concentrée. 21,2 g de poudre blanche sont obtenus avec un rendement de 76 % après chromatographie sur silice (acétate d'éthyle/cyclohexane 1/1).

3) Préparation de la N,N-ditétradécyl-4-{4-[(2,2,2-trifluoro-acétylamino)-méthyl]-[1,3]dioxolan-2-yloxy}-butyramide

3,5 g de 4-hydroxy-N,N-ditétradécyl-butyramide (7,1 mmol) obtenus à l'étape précédente sont mélangés à 1,8 g de 2,2,2-trifluoro-N-(2-méthoxy-[1,3]dioxolan-4-ylméthyl)-acetamide (Ortho 1, 7,8 mmol) et 18 mg de paratoluène sulfonate de pyridinium (PPTS, 0,071 mmol). Le tout est chauffé sans solvant à 80°C pendant 3 heures.
Le brut réactionnel est ensuite dissout dans 200 ml d'heptane, lavé par 3 fois 50 ml d'une solution d'hydrogénocarbonate

de sodium saturée, par 3 fois 50 ml d'acétonitrile puis est concentré à sec.
Une petite fraction du brut est purifiée sur silice pour caractériser cet intermédiaire, le reste étant utilisé tel quel pour l'étape suivante. La chromatographie sur silice (cyclohexane/acétate d'éthyle 7/3 V/V), nous permet d'isoler quelques mg de produit huileux.

4) Préparation de la 4-(4-aminométhyl-[1,3]dioxolan-2-yloxy)-N,N-ditétradécyl-butyramide

Le brut obtenu à l'étape précédente est solubilisé dans 20 ml de tétrahydrofuranne additionnés de 20 ml de soude à 4 %. Le mélange réactionnel est laissé une nuit sous agitation vigoureuse à température ambiante.
Le tétrahydrofuranne est ensuite concentré puis le produit est extrait par 3 fois 200 ml d'éther diéthylique. La phase organique est séchée sur chlorure de calcium, filtrée et évaporée. Une chromatographie sur silice (dichlorométhane/méthanol 9/1 V/V) permet d'isoler 1,6 g d'huile incolore (rendement sur les deux étapes : 38 %).
R.M.N. $^1H$ (300 MHz, $CDCl_3$, $\delta$ en ppm): on observe un mélange de deux diastéréoisomères dans les proportions 50/50.

* 0,89 (t, J = 7 Hz : 6H) ; de 1,15 à 1,45 (mt : 44H) ; 1,52 (mt : 4H) ; 1,58 (mf : 2H) ; 1,95 (quintuplet, J = 6,5 Hz : 2H) ; 2,39 (t, J = 6,5 Hz : 2H) ; de 2,75 à 3,00 (mt : 2H) ; 3,21 (mt : 2H) ; 3,29 (mt : 2H) ; 3,60 (mt : 2H) ; 3,71 et 3,80 (respectivement dd, J = 7,5 Hz et 6 Hz et t, J = 7,5 Hz : 1H en totalité) ; 4,06 et 4,14 (2 t, J = 7,5 Hz : 1H en totalité) ; 4,21 et 4,33 (2 mts : 1H en totalité) ; 5,82 et 5,85 (2 s : 1H en totalité).

**b - Synthèse de l'acide [(3-{bis-[3-(2,2,2-trifluoro-acétylamino)-propyl]-amino}-propyl)-trifluoroacétyl-amino]-acétique sous forme de sel trifluoroacétate (partie hydrophile du type polyamine-COOH)**

La synthèse proposée procède en 6 étapes à partir du 3-aminopropanol et du 3,3'-imino-bispropylamine.

1) Préparation de la 2,2,2-trifluoro-N-{3-[3-(2,2,2-trifluoro-acétylamino)-propylamino]-propyl}-acétamide

35 g de 3,3'-imino-bispropylamine (266,7 mmol) sont solubilisés dans 150 ml de tétrahydrofuranne anhydre sous flux d'argon. Le mélange réactionnel est ensuite refroidi à 0°C par un bain de glace et 65 ml de trifluoroacétate d'éthyle (546,8 mmol) sont ajoutés goutte à goutte (très lentement) par une ampoule de coulée. A la fin de l'addition (3 heures plus tard), on laisse le mélange réactionnel revenir à température ambiante et l'agitation est maintenue quelques heures sous argon.
Le mélange réactionnel est ensuite filtré sur papier. Le filtrat est concentré à sec, repris plusieurs fois dans le dichlorométhane, le séchage final étant réalisé à l'étuve à 40°C sous le vide de la pompe à palette pendant 16 heures. 85,3 g d'une poudre blanche sont isolés purs sans purification supplémentaire (rendement : 99 %).

2) Préparation du (3-hydroxy-propylamino)-acétate de *tert*-butyl

196 ml de 3-aminopropanol (2,56 mol) sont dilués dans 250 ml de dichlorométhane et le tout est refroidi à 0°C par un bain de glace. 20 g de bromoacétate de *tert*-butyl (102,5 mmol) solubilisés dans 200 ml de dichlorométhane sont ensuite additionnés goutte à goutte en maintenant le mélange réactionnel à 0°C. A la fin de l'addition (2 heures plus tard), on laisse le mélange réactionnel à température ambiante pendant 3 heures.
Ce brut est alors lavé par 3 fois 150 ml d'une solution d'hydrogénocarbonate de sodium saturée, puis 3 fois 150 ml d'une solution de chlorure de sodium saturée. La phase organique est séchée sur chlorure de calcium, filtrée et concentrée. On isole ainsi 17,8 g d'une huile incolore (rendement : 92 %).

3) Préparation du [(3-hydroxy-propyl)-trifluoroacétyl-amino]-acétate de *tert*-butyl

17,65 g de (3-hydroxy-propylamino)-acétate de *tert*-butyl (93,3 mmol) sont solubilisés dans 100 ml de dichlorométhane et le tout est refroidi à 0°C par un bain de glace. 26 ml de triéthylamine (186,6 mmol) sont ajoutés, suivis d'une addition goutte à goutte de 21,5 g d'anhydride trifluoroacétique (102,6 mmol) par une ampoule de coulée. A la fin de l'addition, on laisse le mélange réactionnel à température ambiante pendant une nuit sous agitation magnétique.
Cette solution est ensuite lavée par 3 fois 50 ml d'une solution d'hydrogénocarbonate de sodium saturée, 3 fois 50 ml d'une solution d'hydrogénosulfate de potassium 0,5 M, puis 3 fois 50 ml d'une solution de chlorure de sodium saturée.
La phase organique est séchée sur sulfate de magnésium, filtrée et concentrée. On isole ainsi 24,5 g d'une huile jaune pâle (rendement : 92 %).

4) Préparation du [(3-bromo-propyl)-trifluoroacétyl-amino]-acétate de *tert*-butyl

10 g de [(3-hydroxy-propyl)-trifluoroacétyl-amino]-acétate de *tert*-butyl (35 mmol) sont solubilisés dans 150 ml de tétrahydrofuranne. 12,4 g de triphénylphosphine (47,3 mmol) sont ajoutés et le mélange réactionnel est thermostaté à 15-20 °C. 15,1 g de tétrabromure de carbone (45,6 mmol) dissout dans 60 ml d'acétonitrile sont ajoutés goutte à goutte par une ampoule de coulée et le tout est agité à température ambiante pendant 4 heures.
Le mélange réactionnel est alors concentré à sec, repris dans l'acétate d'éthyle et filtré sur papier. Le filtrat est concentré à sec, repris dans le cyclohexane et filtré sur fritté n°3. Le filtrat est de nouveau concentré et purifié par chromatographie sur silice (cyclohexane/acétate d'éthyle 8/2 V/V). 10,4 g d'une huile jaune pâlesont ainsi isolés (rendement : 85 %). ,

5) préparation du [(3-{bis-[3-(2,2,2-trifluoro-acétylamino)-propyl]-amino}-propyl)-trifluoroacétyl-amino]-acétate de *tert*-butyl

26 g de [(3-bromo-propyl)-triffuoroacétyl-amino]-acétate de tert-butyl (74,7 mmol) et 24,1 g de 2,2,2-trifluoro-N-{3-[3-(2,2,2-trifluoro-acétylamino)-propylamino]-propyl}-acétamide (74,7 mmol) sont solubilisés dans 130 ml d'acétonitrile. 30 g de carbonate de potassium (224 mmol) sont alors mis en suspension et le tout est chauffé au reflux pendant 6 heures.
Le mélange réactionnel est alors filtré sur papier et concentré à sec. Le brut est ensuite purifié par chromatographie sur silice (cyclohexane/acétate d'éthyle 2/8 V/V). 16,6 g d'une huile jaune pâle (rendemnt : 38 %) sont ainsi isolés.

6) préparation du sel trifluoroacétate d'acide [(3-{bis-[3-(2,2 2-trifluoro-acétylamino)-propyl]-amino}-propyl)-trifluoroacétyl-amino]-acétique

15,8 g de [(3-{bis-[3-(2,2,2-trifluoro-acétylamino)-propyl]-amino}-propyl)-trifluoroacétyl-amino]-acétate de tert-butyl (28,76 mmol) sont additionnés de 50 ml de dischlorométhane puis de 50 ml d'acide trifluoroacétique. Ce mélange est agité quelques heures à température ambiante. Le mélange réactionnel est alors concentré à sec. 18,7 g de miel jaune pâle sont ainsi isolés(rendement : 100%).
R.M.N. $^1H$ (300 MHz, $CDCl_3$, δ en ppm) : à température ambiante, on observe un mélange de rotamères.

* de 1,80 à 2,10 (mt : 6H) ; 3,12 (mt : 6H) ; 3,29 (mt : 4H) ; 3,50 (mt : 2H) ; 4,13 et 4,29 (respectivement s large et mt : 2H en totalité) ; de 9,50 à 9,75 (mt : 2H)

**c - Synthèse du tétrachlorhydrate de 4-{4-[(2-{3-[bis-(3-amino-propyl)-amino]-propylamino}-acétylamino)-méthyl]-[1,3]dioxolan-2-yloxy}-N,N-ditétradécyl-butyramide (composé B)**

Cette synthèse procède en trois étapes : condensation des deux molécules obtenues aux parties a et b ci-avant, puis déprotection de la polyamine et salification.

1) Préparation de la 4-[4-({2-[(3-{bis-[3-(2,2,2-trifluoro-acétylamino)-propyl]-amino}-propyl)-trifluoroacétyl-amino]-acétylamino}-méthyl)-[1,3]dioxolan-2-yloxy]-N,N-ditétradécyl-butyramide

A 1,65 g de 4-(4-aminométhyl-[1,3]dioxolan-2-yloxy)-N,N-ditétradécyl-butyramide (2,76 mmol) dissout dans 30 ml de dichlorométhane sont additionnés successivement 1.9 ml de triéthylamine (13,8 mmol), 2 g d'acide [(3-{bis-[3-(2,2,2-trifluoro-acétylamino)-propyl]-amino}-propyl)-trifluoroacétyl-amino]-acétique (sel trifluoroacétate, 3,04 mmol) et 1,8 g de BOP (4,14 mmol). La solution est agitée 1 heure à température ambiante.
Le brut réactionnel est alors concentré à sec, repris dans 200 ml d'acétate d'éthyle, lavé par 40 ml d'une solution de chlorure de sodium saturée, puis 3 fois 40 ml d'une solution d'hydrogénocarbonate de sodium saturée, puis 3 fois 40 ml d'une solution de chlorure de sodium saturée. Le produit est ensuite purifié par chromatographie sur silice (Elution : acétate d'éthyle). 2,2 g de miel jaune pâle sont ainsi isolés (rendement : 72 %).

2) Préparation de la 4-{4-[(2-{3-[bis-(3-amino-propyl)-amino]-propylamino}-acétylamino)-méthyl]-[1,3]dioxolan-2-yloxy}-N,N-ditétradécyl-butyramide

2,1 g de 4-[4-({2-[(3-{bis-[3-(2,2,2-trifluoro-acétylamino)-propyl]-amino}-propyl)-trifluoroacétyl-amino]-acétylamino}-méthyl)-[1,3]dioxolan-2-yloxy]-N,N-ditétradécyl-butyramide (1,88 mmol) sont dissout dans 30 ml de tétrahydrofuranne, et 30 ml de soude molaire à 4 % sont additionnés sous forte agitation. La réaction est laissée une nuit à température ambiante.
Le solvant est ensuite concentré puis le brut est repris dans un mélange dichlorométhane/méthanol 1/1. Cette solution

brute est purifiée par chromatographie sur silice (dichlorométhane/méthanol/ammoniac, 45/45/10, V/V/V). Le produit est concentré puis lyophilisé après adjonction d'eau. 1,3 g de lyophilisât blanc sont ainsi obtenus (rendement : 84 %).

3 Préparation du tétrachlorhydrate de 4-{4-[(2-{3-[Bis-(3-amino-propyl)-amino]-propylamino}-acétylamino)-méthyl]-[1,3]dioxolan-2-yloxy}-N,N-ditétradécyl-butyramide (composé B)

Le produit obtenu à l'étape précédente sous forme de base libre est ensuite salifié quantitativement sur résine échangeuse d'ions : il est solubilisé dans l'eau, et élué dans une colonne contenant un large excès de résine chlorure (FLUKA ; DOWEX 21K). La structure du lyophilisât blanc obtenu est confirmée par RMN $^1H$.
R.M.N. $^1H$ (300 MHz, $(CD_3)_2SO$ d6, $\delta$ en ppm) : on observe un mélange de deux diastéréoisomères dans les proportions 50/50.

* 0,89 (t, J = 7 Hz : 6H) ; de 1,15 à 1,40 (mt : 44H) ; de 1,40 à 1,60 (mt : 4H) ; 1,72 (mt : 8H) ; 2,31 (mt : 2H) ; de 2,40 à 2,55 (mt : 6H) ; de 2,70 à 2,90 (mt : 6H) ; de 3,15 à 3,75 - de 4,00 à 4,40 (2 séries de mt : 13H en totalité) ; 5,83 et 5,86 (2 s : 1H en totalité).

**EXEMPLE 5 : Synthèse des lipides PEGoylés C et D**

Cet exemple décrit une voie de synthèse des lipides pégoylés Octadécanol-Ortho1-$PEG_{5000}$-OMe et Cholestérol-Ortho1-$PEG_{5000}$-OMe qui ne diffèrent l'un de l'autre que de par leur partie lipidique : octadécanol pour le composé C et cholestérol pour le composé D. Ces deux composés acidosensibles ont pour formule générale :

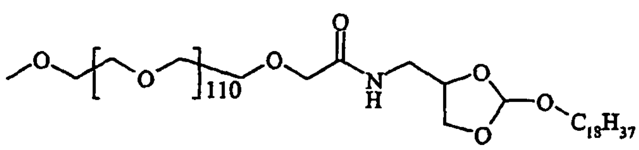

Composé C

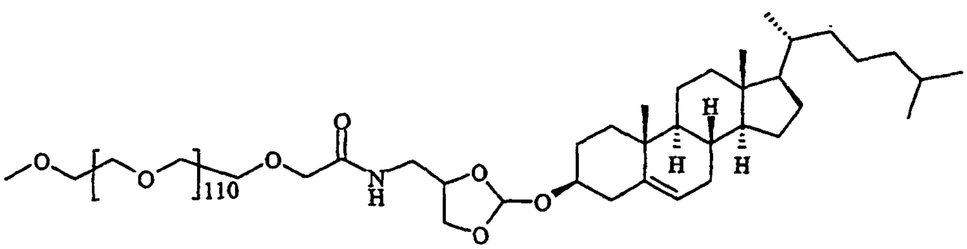

Composé D

**a - Synthèse de la C-(2-octadécyloxy-[1,3]dioxolan-4-yl)-méthylamine et de la C-{2-[17-(1,5-Diméthyl-héxyl)-10,13-diméthyl-2,3,4,7,8,9,10,11,12,13,14,15,16,17-tetradécahydro-1H-cyclopenta[a]phénanthrèn-3-yloxy]-[1,3]dioxolan-4-yl}-méthylamine (parties lipide-O-Ortho1-$NH_2$)**

Cette synthèse procède en deux étapes à partir du composé Ortho 1 par substitution du groupe méthoxy exocyclique par l'alcool gras (cholestérol ou octadécanol) puis déprotection de l'amine.

1) Préparation de la 2,2,2-trifluoro-N-(2-octadécyloxy-[1,3]dioxolan-4-ylmethyl)-acétamide

3 g de 2,2,2-trifluoro-N-(2-méthoxy-[1,3]dioxolan-4-ylméthyl)-acétamide (Ortho 1, 13,09 mmol) sont mélangés à 3,54 g d'octadécanol (13,09 mmol). Le mélange est mis en fusion à 80°C et laissé pendant 2 heures après l'ajout de 32 mg de paratoluène sulfonate de pyridinium (0,13 mmol). Le brut réactionnel est ensuite dissout dans du cyclohexane, lavé par une solution d'hydrogénocarbonate de sodium saturée puis par une solution de chlorure de sodium saturée, séché sur sulfate de magnésium puis concentré à sec. Ce brut est utilisé tel quel pour l'étape suivante.

1') Préparation de la N-{2-[17-(1,5-diméthyl-hexyl)-10,13-diméthyl-2,3,4,7,8,9,10,11,12,13,14,15,16,17-tetradécahydro-1H-cyclopenta[a]phénanthrèn-3-yloxy]-[1,3]dioxolan-4-ylméthyl}-2,2,2-trifluoro-acétamide

Le protocole est identique à celui décrit précédemment, la réaction pouvant aussi être réalisée sans catalyseur.

2) Préparation de la C-(2-octadécyloxy-[1,3]dioxolan-4-yl)-méthylamine

6,12 g de brut réactionnel de l'étape 1 précédente (13,09 mmol) sont dissout dans 20 ml de tétrahydrofuranne. Le mélange réactionnel est refroidit par un bain de glace, et 30 ml de soude à 4 % sont additionnés. Le mélange est agité à température ambiante jusqu'à disparition totale du réactif (pendant 4 heures).
Ensuite, le solvant est concentré en partie puis extrait par 3 fois 200 ml d'éther diéthylique. La phase organique est séchée sur chlorure de calcium, filtrée et évaporée. Le brut réactionnel est purifié par chromatographie sur silice (dichlorométhane/méthanol 9/1, V/V). 2,6 g de poudre blanche sont ainsi recueillis (rendement : 53 % sur les deux étapes 1 et 2 consécutives).
R.M.N. $^1H$ (300 MHz, $CDCl_3$, $\delta$ en ppm). On observe un mélange de deux diastéréoisomères dans les proportions approximatives 50/50.

* 0,89 (t, J = 7 Hz : 3H) ; de 1,20 à 1,45 et 1,58 (2 mts : 32H en totalité) ; de 2,75 à 3,00 (mt : 2H) ; 3,53 (mt : 2H) ; de 3,65 à 3,85 (mt : 1H) ; de 4,00 à 4,40 (mt : 2H) ; 5,81 et 5,84 (2s : 1H en totalité).

2') Préparation de la C-{2-[17-(1,5-Diméthyl-héxyl)-10,13-diméthyl-2,3,4,7,8,9,10, 11,12,13,14,15,16,17-tetradécahydro-1H-cyclopenta[a]phénanthrèn-3-yloxy]-[1,3] dioxolan-4-yl}-méthylamine

Le protocole est identique à celui de l'étape 2). 1,4 g de poudre blanche sont ainsi recueillis (rendement : 22 % sur les deux étapes 1' et 2' consécutives).
R.M.N. $^1H$ (400 MHz, $CDCl_3$, $\delta$ en ppm). On observe un mélange de deux diastéréoisomères dans les proportions approximatives 50/50.

* 0,69 (s : 3H) ; de 0,85 à 1,75 - 1,86 et 2,00 (mts : 26H en totalité) ; 0,88 (mt : 6H) ; 0,93 (d, J = 7 Hz : 3H) ; 1,01 (s : 3H) ; 2,33 (mt : 2H) ; de 2,75 à 3,00 (mts : 2H) ; 3,50 (mt : 1H) ; 3,71 - 3,85 - 4,05 et 4,16 (4 mts : 2H en totalité) ; 4,20 et 4,35 (2 mis : 1H en totalité) ; 5,36 (mt : 1H) ; 5,93 et 5,96 (2s : 1H en totalité).

**b - Synthèse de l'acide du méthoxy-polyéthylène glycol 5000 (partie hydrophile MeO-$PEG_{5000}$-COOH)**

Une seule étape est nécessaire : oxydation du groupe hydroxyle terminal du méthoxy-polyéthylène glycol commercial.
20 g de MeO-$PEG_{5000}$-OH (4 mmol) sont dissout dans 100 ml d'un mélange équivolumique eau / acétonitrile. 312 mg de 2,2,6,6-tétraméthyl-pipéndinyloxyl (2 mmol) puis 6,4 g de [bis-(acétoxy)-iodo]benzène (20 mmol) sont additionnés et le mélange réactionnel est laissé sous agitation pendant 16 heures à température ambiante.
Ce brut réactionnel est ensuite évaporé à sec, repris dans 40 ml d'un mélange dichlorométhane/éthanol (1/1 ; V/V), puis précipité par adjonction de 500 ml d'éther diéthylique. 19 g de poudre blanche sont ainsi isolés par filtration et lavage à l'éther (rendement : 95 %).
R.M.N. $^1H$ (300 MHz, $CDCl_3$, $\delta$ en ppm) : 3,39 (s : 3H) ; de 3,40 à 3,95 (mts : 404H); 4,15 (s : 2H).

**c - Synthèse de la méthoxy-(polyéthylène glycol 5000)-N-(2-octadécyloxy-[1,3]dioxolan-4-ylméthyl)-amide (composé C) et de la méthoxy-(polyéthylène glycol 5000)-N-{2-[17-(1,5-Diméthyl-hexyl)-10,13-diméthyl-2,3,4,7,8,9,10,11, 12,13,14,15,16,17-tétradécahydro-1H-cyclopenta[a]phénanthrèn-3-yloxy]-[1,3]dioxolan-4-ylméthyl}-amide (composé D)**

Composé C :

1,2 g de MeO-$PEG_{5000}$-COOH (0,24 mmol) obtenus lors des étapes précédentes sont dissout dans 5 ml de dichlorométhane. 188 µl de triéthylamine (1,34 mmol) sont ajoutés puis 100 mg de C-(2-octadécyloxy-[1,3]dioxolan-4-yl)-méthylamine (0,27 mmol). 143 mg de BOP (0,32 mmol, 1.2eq) sont alors ajoutés et la réaction est laissée sous agitation à température ambiante pendant une heure.
Le mélange réactionnel est précipité par adjonction d'éther diéthylique (60 ml), centrifugé, lavé à l'éther puis injecté en Chromatographie Liquide Haute performance (CLHP) préparative. En isolant les fractions les plus pures, 415 mg

de lyophilisât blanc sont ainsi obtenus (rendement : 32 %).
R.M.N. $^1$H (300 MHz, $CDCl_3$, δ en ppm) : on observe un mélange de deux diastéréoisomères dans les proportions approximatives 50/50.

* 0,90 (t, J = 7 Hz : 3H) ; de 1,20 à 1,40 et de 1,50 à 1,75 (mts : 32H) ; 3,39 (s : 3H) ; de 3,40 à 3,95 (mts : 448H) ; 4,02 et 4,03 (2s : 2H en totalité) ; de 4,00 à 4,25 (mts : 3H en totalité) ; 5,80 et 5,84 (2s : 1H en totalité).

Composé D :

Le protocole est identique à celui exposé pour la préparation du composé C. En isolant les fractions les plus pures, 395 mg de lyophilisât blanc sont ainsi obtenus (rendement : 30 %).
R.M.N. $^1$H (400 MHz, $CDCl_3$, δ en ppm) : on observe un mélange de deux diastéréoisomères dans les proportions approximatives 50/50.

* 0,68 (s : 3H) ; de 0,85 à 1,75 - 1,85 et 2,00 (mts : 26H en totalité) ; 0,87 (mt : 6H) ; 0,92 (d, J = 7 Hz : 3H) ; 1,00 (s : 3H) ; 2,33 (mt : 2H) ; 3,39 (s : 3H) ; de 3,40 à 3,90 (mts : 448H) ; de 4,00 à 4,20 (mts : 1H en totalité) ; 4,01 et 4,04 (2s : 2H en totalité) ; 4,28 et 4,46 (2 mts : 1H en totalité) ; 5,35 (mt : 1H) ; 5,90 et 5,95 (2s : 1H en totalité).

**EXEMPLE 6 : Synthèse du lipide PEGoylé E**

Cet exemple décrit une voie de synthèse du lipide pégoylé Octadécanol-Ortho2-$PEG_{5000}$-OMe qui a pour formule générale :

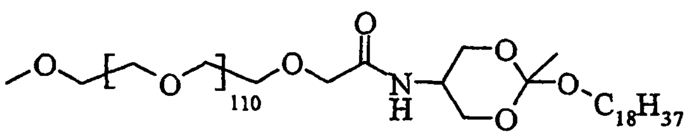

Composé E

**a - Synthèse de la 2-méthyl-2-octadécyloxy-[1,3]dioxan-5-ylamine (partie hydrophobe lipide-O-Ortho2-$NH_2$)**

Cette synthèse procède en deux étapes à partir du substrat Ortho 2 dont la synthèse est décrite ci-avant, par substitution du groupe méthoxy exocyclique par l'octadécanol puis déprotection de l'amine.

1) Préparation de la 2,2,2-trifluoro-N-(2-méthyl-2-octadécyloxy-[1,3]dioxan-5-yl)-acétamide

3 g de 2,2,2-trifluoro-N-(2-méthoxy-2-méthyl-[1,3]dioxan-5-yl)-acétamide (12,34 mmol) sont mélangés à 3 g d'octadécanol (11,1 mmol). Le mélange est mis en fusion à 80°C et laissé pendant 2 heures pour évaporer tout le méthanol produit lors de l'échange d'alcool.
Le mélange réactionnel en fusion est ensuite versé dans 50 ml d'acétonitrile ce qui induit sa précipitation. Le produit attendu est purifié par recristallisation dans l'acétonitrile. 2,85 g de poudre blanche sont ainsi isolés (rendement : 53 % après recristallisation).

2) Préparation de la 2-méthyl-2-octadécyloxy-[1,3]dioxan-5-ylamine

1 g du dérivé trifluoroacétamide de l'étape précédente (2,08 mmol) est dissout dans 10 ml de tétrahydrofuranne auxquels on additionne 10 ml de soude molaire à 4 %. Le mélange est agité vigoureusement à température ambiante jusqu'à disparition totale du réactif (pendant 4 heures).
Ensuite, le solvant est concentré en partie puis extrait par 3 fois 100 ml d'éther diéthylique. La phase organique est séchée sur chlorure de calcium, filtrée et évaporée à sec. Ainsi, 810 mg de poudre blanche sont isolés sans purification (rendement : 100%).
R.M.N. $^1$H (400 MHz, $CDCl_3$, δ en ppm) : 0,89 (t, J = 7 Hz : 3H) ; de 1,20 à 1,50 (mt : 30H) ; 1,49 (s : 3H) ; 1,62 (mt : 2H) ; 1,67 (s large : 2H) ; 2,71 (mt : 1H) ; 3,46 (t, J = 7 Hz : 2H) ; 3,54 (d large, J = 10 Hz : 2H) ; 4,30 (dd, J = 10 et 1,5 Hz : 2H).

**b - Synthèse de la méthoxy-(polyéthylène glycol 5000)-N-(2-méthyl-2-octadécyloxy-[1,3]dioxan-5-yl)-amide (Composé E)**

La dernière étape consiste à condenser l'amine lipidique sur l'acide du PEG (dont la synthèse est décrite dans l'exemple 5).

1,15 g de MeO-$PEG_{5000}$-COOH (0,23 mmol) sont dissout dans 5 ml de dichlorométhane. 181 µl de triéthylamine (1,30 mmol) sont ajoutés puis 100 mg de 2-méthyl-2-octadécyloxy-[1,3]dioxan-5-ylamine (0,26 mmol). 172 mg de BOP (0,39 mmol) sont alors ajoutés et la réaction est laissée sous agitation à température ambiante pendant une heure.

Le mélange réactionnel est précipité par adjonction d'éther diéthylique (60 ml), centrifugé, lavé à l'éther puis injecté en CLHP préparative. En isolant les fractions les plus pures, 420 mg de lyophilisât blanc sont ainsi obtenus (rendement : 34 %).

R.M.N. $^1H$ (400 MHz, $CDCl_3$, $\delta$ en ppm) : 0,89 (t, J = 7 Hz : 3H); de 1,20 à 1,50 (mt : 30H) ; 1,48 (s : 3H) ; de 1,55 à 1,75 (mt : 2H) ; 3,39 (s : 3H) ; de 3,40 à 3,90 (mt : 448H) ; 3,89 (d large, J = 8,5 Hz : 1H) ; 4,05 (s : 2H) ; 4,30 (d large, J = 12 Hz : 2H) ; 7,57 (d, J = 8,5 Hz : 1H).

**EXEMPLE 7 : Etude de la compaction de l'ADN par les composés acido-sensibles A Syn et Trans**

Les composés acidosensibles A formes Syn et Trans préparés ci-avant ont une structure analogue aux lipides cationiques utilisés classiquement pour la transfection non-virale d'ADN, et ils possèdent en outre dans leur structure une fonction orthoester cyclique qui contribue à les rendre acidosensibles.

Le but de cet exemple est donc de démontrer que les composés acidosensibles A Syn et Trans conservent le pouvoir de compacter l'ADN à transfecter propre aux lipides cationiques, tout en ayant la capacité de se dégrader en milieu acide et donc de libérer l'ADN compacté. Ceci peut être aisément montré par un test de fluorescence au bromure d'éthidium (BET) : l'absence de fluorescence témoigne de l'absence d'ADN libre ce qui signifie que l'ADN est compacté.

Dans toute la suite, les deux formes Syn et Trans du composé acidosensible A tel que préparé dans l'exemple 3 ont été utilisés et l'analogue non-acidosensble décrit dans la publication WO 97/18185, et de formule :

$$H_2N\text{-}(CH_2)_3\text{-}NH\text{-}(CH_2)_4\text{-}NH\text{-}(CH_2)_3\text{-}NH\text{-}CH_2\text{-}CO\text{-}Gly\text{-}N[(CH_2)_{17}\text{-}CH_3]_2$$

a été utilisé comme témoin. Cet analogue non-acidosensible est dénommé dans toute la suite « lipide cationique témoin ».

L'ADN est mis en présence de quantités croissantes de lipide cationique témoin ou de composé acidosensible A Syn ou Trans, par mélange équivolumétrique de solutions lipidiques de différents titres dans les solutions d'ADN. On prépare ainsi des échantillons de 800 µl de complexes d'ADN de concentration 10 µg/ml dans une solution de chlorure de sodium à 150 mM avec des quantités croissantes de lipide cationique témoin ou de composé acidosensible A Syn ou Trans .

Au temps t = 0, on ajoute à ces échantillons 200 µl de tampon de concentration 0,1 mol/l à pH 5, et les échantillons sont stockés dans une étuve à 37°C. La fluorescence au bromure d'éthidium (BET) est mesurée au fil du temps (mesure à 20°C) en utilisant un FluoroMax-2 (Jobin Yvon-Spex), avec des longueurs d'onde d'excitation et d'émission respectivement de 260 nm et 590 nm. Les largeurs de fentes pour l'excitation et l'émission sont réglées à 5 nm. La valeur de fluorescence est enregistrée après ajout de 3 µl de bromure d'éthidium à 1 g/l par ml de solution ADN/lipide cationique ou ADN/composé acidosensible (à 0,01 mg d'ADN/ml).

Les résultats sont résumés dans la figure 1. A pH 5, lorsque la quantité de composé acidosensible A Syn ou Trans ou de lipide cationique témoin utilisée pour compacter l'ADN est trop faible (0,4 nmol lipide / µg d'ADN) et que l'ADN n'est donc pas complètement compacté, aucune évolution significative de la fluorescence n'est mesurable au fil du temps. En revanche, on observe un comportement différent des composés acidosensibles A Syn et Trans par rapport au lipide cationique témoin (non-acidosensible) pour des quantités plus importantes (1,7 nmol lipide / µg d'ADN et 6,0 nmol lipide / µg d'ADN) qui permettent la compaction totale de l'ADN. En effet, les composés acidosensibles A Syn et Trans libèrent l'ADN au cours du temps comme en témoigne l'augmentation de fluorescence, ce qui n'est pas le cas avec le lipide cationique témoin qui n'est pas acidosensible. On constate en outre que cette libération de l'ADN intervient quelques heures après l'addition d'acide (à pH 5 et 37°C).

On observe également un décalage dans la cinétique de libération de l'ADN selon la quantité de composé acidosensible utilisée : la libération de l'ADN est d'autant plus rapide que la quantité de composé acidosensible A utilisée est faible.

Cette étude démontre les propriétés remarquables des composés acidosensibles A Syn et Trans : ils sont capables de former des complexes avec l'ADN en compactant celui-ci, et leur dégradation en milieu acide entraîne une dégradation des complexes formés avec l'ADN, et donc la libération de l'ADN. Ces composés acidosensibles sont

donc particulièrement utiles dans le cadre de la transfection non-virale d'ADN dans les cellules.

**EXEMPLE 8 : Etude du pouvoir transfectant des composés acido-sensibles A Syn et Trans *in vitro*.**

Cet exemple illustre le pouvoir *transfectant in vitro* des composés acidosensibles A Syn et Trans, comparativement à leur analogue non-acidosensible décrit à l'exemple précédent (le lipide cationique témoin).

Cette étude a été menée à 4 rapports de charge différents : 1,0 ou 4,0 ou 6,0 ou 10,0 nmol de lipide / µg d'ADN. Chacune de ces conditions a été testée avec et sans sérum de veau foetal (« + ou - Sérum »)

Culture des cellules : des cellules HeLa (American type Culture Collection (ATCC) Rockville, MD, USA) dérivées d'un carcinome d'epithélium cervical humain, sont mises en culture en présence d'un milieu de type MEM («minimum essential medium ») avec addition de 2 mM de L-glutamine, 50 unités/ml de penicilline, et 50 unités/ml de streptomycine. Le milieu et les additifs proviennent de Gibco/BRL life Technologies (Gaithersburg, MD,USA). Les cellules sont cultivées en flasks à 37°C et à 5 % de dioxyde de carbone en incubateur.

Transfection : un jour avant la tranfection, les cellules HeLa sont tranférées dans des plaques 24 puits avec un nombre de cellules de 30000 à 50000 par puits. Ces dilutions représentent approximativement 80% de confluence après 24 heures. Pour la transfection, les cellules sont lavées deux fois et incubées à 37°C avec 500 µl de milieu avec serum (10 % SVF v/v) ou sans sérum.
50 µl de complexes contenant 0,5 µg d'ADN plasmidique sont ajoutés dans chaque puit (les complexes sont préparés au moins 30 minutes avant l'ajout dans les puits). Après deux heures à 37°C les plaques sans serum sont complémentés avec 10 % (v/v) de SVF (« Sérum de Veau Foetal »).
L'ensemble des plaques est placé 36 heures à 37°C et à 5 % de dioxyde de carbone. Détermination de l'activité luciférase : Brièvement, les cellules tranfectées sont lavées deux fois avec 500 µl de PBS (tampon phosphate) puis lysées avec 250 µl de réactif (Promega cell culture lysis reagent, du kit Luciferase Assay System).
Un aliquot de 10 µl de surnageant du lysat centrifugé (12000 x g) 5 minutes à 4°C est mesuré au luminomètre Wallac Victor2 (1420 Multilabel couter).
L'activité luciférase est dosée par l'émission de lumière en présence de luciférine, de coenzyme A et d'ATP pendant 10 secondes et rapportée à 2000 cellules traitées. L'activité luciférase est ainsi exprimée en Unité de Lumière Relative (« RLU » : «Relative light unit ») et normalisée avec la concentration de proteines de l'échantillon obtenue par l'utilisation d'un kit Pierce BCA (Rockford, IL, USA).

Les résultats résumés à la figure 2 montrent une activité transfectante importante pour les trois composés testés (les composés acidosensibles A Syn et Trans et le lipide cationique témoin). On n'observe aucune différence significative entre eux. En l'absence de sérum, le niveau de transfection est élevé dans tous les cas ($10^5$ à $10^7$ RLU/µg de protéine) et le pouvoir transfectant augmente avec la quantité de composé acidosensible ou de lipide cationique témoin utilisée. La présence de sérum induit une inhibition de la tranfection dans tous les cas.

Cette exemple montre donc que le pouvoir transfectant du composé acidosensible A sous ses formes Syn et Trans est conservé comparativement à son analogue non-acidosensible (le lipide cationique témoin). Plus généralement, l'introduction d'une fonction orthoester cyclique acidosensible dans des molécules de type lipide cationique connues pour être utiles en transfection non-virale ne détruit pas la capacité de ces composés à transfecter efficacement l'ADN.

**EXEMPLE 9 : Utilisation des composés acidosensibles C et D en tant qu'agents de surface non-ioniques pour la stabilisation colloïdale de complexes transfectants ADN/lipide cationique**

Cet exemple illustre le fait que les lipides pégoylés acidosensibles du type de ceux définis sous (d) dans la présente demande peuvent être utilisés en tant qu'agents de surface non-ioniques qui jouent un rôle de stabilisateur colloïdal vis-à-vis des particules transfectantes ADN/lipide cationique.

Dans le présent exemple, le lipide cationique utilisé est celui déjà utilisé dans les exemples 7 et 8 et décrit dans la publication WO 97/18185 sous la formule :

$$H_2N\text{-}(CH_2)_3\text{-}NH\text{-}(CH_2)_4\text{-}NH\text{-}(CH_2)_3\text{-}NH\text{-}CH_2\text{-}CO\text{-}Gly\text{-}N[(CH_2)_{17}\text{-}CH_3]_2$$ (lipide cationique témoin).

Les composés C et D préparés à l'exemple 5 sont utilisés comme lipides pegoylés acidosensibles. A titre de témoins, on utilise le BRIJ 700 (SIGMA) et le lipide pegoylé de formule :

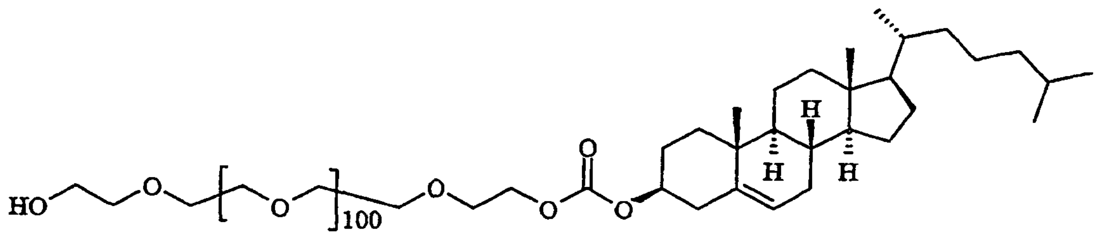

"Analogue D"

qui sont les analogues non-acidosensibles respectivement des composés C et D, et qui sont connus en tant qu'agents de surface non-ioniques (voir par exemple la publication WO 98/34648). Les deux témoins sont utilisés à 10 g/l dans l'eau.

Des échantillons de 1 ml de complexes nucléolipidique (ADN/lipide cationique témoin) sont préparés à partir d'ADN à 10 µg/ml dans 75 mM d'une solution de chlorure de sodium, par mélange équivolumétrique de la solution contenant le lipide cationique témoin et un des lipides pegoylés (le composé C ou le composé D ou le Brij 700 ou l'analogue D) dans la solution d'ADN. Ces échantillons ont tous un rapport lipide cationique témoin/ADN de 1,5 (en nmol de lipide par µg d'ADN) et contiennent des quantités croissantes de lipide pégoylé (exprimées en rapport poids/ poids polymère/ADN).

La mesure de la taille des particules obtenue est faite 30 minutes après le mélange et permet notamment d'étudier l'influence de la quantité de lipide pégoylé acidosensible (composé C ou D) ou de lipide pegoylés non-acidosensibles (Brij 700 ou analogue D) sur la stabilisation des complexes lipide cationique témoin/ADN.
La mesure du diamètre hydrodynamique est réalisée avec un appareil Coulter N4Plus, en utilisant des cuves plastiques (quatre faces transparentes) remplies avec 800 µl des différentes solutions à 0,01 mg d'ADN/ml, la mesure étant réalisée à 90° en mode unimodale.

Les résultats sont présentés à la figure 3 qui décrit l'évolution de la taille des particules lipide cationique témoin/ADN en fonction de la quantité de composé C ou D ou de Brij 700 ou d'analogue D utilisée.
On observe que les lipides pegoylés acidosensibles, aussi bien que leur témoins stables, permettent la formation de particules lipide cationique témoin/ADN de petite taille (inférieure à 100 nm) quand une quantité minimale est atteinte, alors que ces mêmes particules lipide cationique témoin/ADN s'agrègent en spontanément (taille supérieure à 1 µm) en l'absence de lipide pegoylé acidosensibles ou non-acidosensibles ou lorsque celui-ci est en trop faible quantité.

Cet exemple démontre ainsi que les composés C et D, et plus généralement les lipides pégoylés acidosensibles du type de ceux définis sous (d) dans la formule générale (I) de la présente demande, peuvent être utilisés en tant qu'agents de surface non-ioniques, et leur pouvoir stabilisateur colloïdal est comparable à celui des agents de surface non-ioniques stables (c'est-à-dire non-acidosensbles tels que le Brij 700 par exemple) classiquement utilisés pour la stabilisation colloïdale de particules nucléolipidiques.

**EXEMPLE 10 : Influence du pH sur la stabilisation colloïdale des complexes ADN/vecteur cationique par les composés C et D**

Cet exemple illustre le fait que lipides pégoylés acidosensibles du type de ceux définis sous (d) dans la formule générale (I) de la présente demande, et utilisés en tant qu'agents de surface non-ioniques pour stabiliser des complexes nucléolipidiques ADN/lipide cationique, peuvent être dégradés à pH acide et ainsi libérer lesdits complexes nucléolipidiques.

La propriété remarquable qui est utilisée ici est l'absence de stabilisation colloïdale lorsqu'on remplace un lipide pégoylé par un PEG sans partie lipidique. En effet, la formulation de l'ADN en présence d'un vecteur cationique et d'un lipide pegoylé acidosensible conduit dans un milieu non tamponné à des petites particules (voir exemple 9 ci-avant). La même étude avec un PEG seul (c'est-à-dire non couplé à une partie lipidique) ne donne en revanche aucune stabilisation.

Le lipide pegoylé acidosensible utilisé dans cet exemple est le composé D préparé à l'exemple 5, ainsi que son analogue non-acidosensible appelé « Analogue D » dans l'exemple précédent et dans toute la suite.

Des échantillons de 1 ml de complexes nucléolipidique (ADN/lipide cationique témoin) sont préparés à partir d'ADN à 10 µg/ml dans 75 mM d'une solution de chlorure de sodium, par mélange équivolumétrique de la solution contenant le lipide cationique témoin et le composé D ou l'analogue D non-acidosensible dans la solution d'ADN. Ces échantillons ont tous un rapport lipide cationique témoin/ADN de 1,5 (en nmol de lipide par µg d'ADN) et contiennent

des quantités croissantes de lipide pégoylé (exprimées en rapport poids/poids polymère/ADN).
100 µl de tampon acide acétique / acétate de sodium à pH 5 (0,1 mol/l) sont ajoutés à ces échantillons thermostatés à 37°C dans une étuve ventilée. La taille des particules est mesurée en fonction du temps.

Les résultats obtenus sont représentés à la figure 4 qui représente la taille des particules nucléolipidiques en fonction du temps, et également selon le rapport lipide pegoylé acidosensible ou non-acidosensible/ADN (3 rapports poids/poids testés : 0,5 ou 0,75 ou 1)
Dans le cas de l'analogue D (lipide pegoylé non-acidosensible), le pH est sans influence sur la stabilité colloïdale des particules : les particules formées ont une petite taille, de l'ordre de 100 nm, quelque soit le rapport analogue D/ADN utilisé. Au temps t = 0, la même stabilité est observée avec le composé D, quelque soit le rapport composé D/ADN.
En revanche, on constate une augmentation de la taille des particules nucléolipidiques en fonction du temps lorsqu'on utilise le composé D comme agent de surface non-ionique à pH acide (pH 5). Cette augmentation de la taille des particules nucléolipidiques est d'autant plus rapide que le rapport composé D/ADN est faible. Ainsi, après 4 heures, les particules sont totalement agrégées dans le cas d'un faible rapport (de 0,5) et pas ou peu lorsqu'un fort excès de composé D est utilisé (rapport de 1).

Il est ainsi possible d'en déduire que le composé D est sensible à la valeur du pH. En effet, l'augmentation de la taille des particules nucléolipidiques en fonction du temps témoigne de la dégradation du lipide pegoylé acidosensible (composé D) lorsqu'on se place en milieu acide (il y a en fait « dégreffage » de la partie lipidique au niveau de la partie acidosensible du composé).
En outre, en augmentant le rapport lipide pegoylé acidosensible/ADN, on augmente aussi le temps nécessaire à l'agrégation, ce qui tend à montrer que plus on utilise de lipide pegoylé acidosensible, plus il y en a à dégrader avant de franchir le seuil au delà duquel il se produit une agrégation des particules nucléolipidiques. Il est ainsi possible, en adaptant la quantité de stabilisateur colloïdal acidosensible, de programmer le temps nécessaire à la libération du principe actif à un pH donné.

**EXEMPLE 11 :Transfection *in vivo* d'ADN formulé avec un mélange lipide cationique/co-lipide neutre/composé D**

Cet exemple illustre l'impact d'un agent de surface non-ionique tel que le composé D sur l'efficacité de transfert d'ADN formulé avec un liposome à base de lipide cationique.

1) Préparation des liposomes

Le lipide cationique de formule :

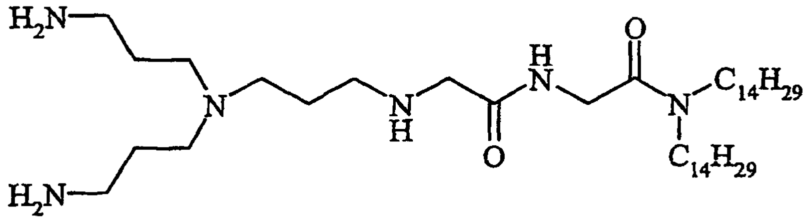

et le lipide neutre DOPE (obtenu de Avanti Polar Lipids, Birmingham, AL) ont chacun été dissout dans du chloroforme puis mélangés en quantités équimolaires.

Des quantités appropriées de composé D ont été dissoutes dans du chlororforme et ajoutées au mélange. Les quantités de composé D utilisées sont données en % molaire de la quantité totale de lipide.

Le solvant organique est ensuite évaporé sous flux d'argon de façon à ce qu'il se forme un film lipidique fin dans le fond du tube. Ce film est séché sous vide pendant au moins 1 heure puis réhydraté avec un tampon HEPES 20 mM à pH 7,4 et du dextrose 5%, à 4°C pendant 2 heures. La suspension lipidique ainsi obtenue est chauffée à 50°C pendant 30 minutes, puis soniquée pendant 5 minutes de façon à former une suspension homogène de liposomes d'environ 100 nm.

L'ADN utilisé est un ADN plasmidique contenant le gène CAT sous contrôle d'un promoteur CMV. L'ADN plasmidique utilisé possède les critères suivants : niveau d'endotoxines inférieur à 20 U/mg, quantité d'ADN superenroulé supérieure à 90%, contamination par l'ADN de *E. coli* inférieure à 5% contamination par l'ARN inférieure à 5% et contamination par des protéines inférieure à 1%.

2) Préparation des complexes ADN/lipides

Des volumes équivalents de suspension de liposomes (en concentration appropriée) sont rapidement ajoutés à une solution d'ADN tout en mélangeant rapidement et vigoureusement. Globalement, 1 µg d'ADN se complexe avec 5 nmol de lipide cationique. Les complexes formés ont approximativement 100 à 240 nm de diamètre.

3) Administration *in vivo*

Des souris femelles Balb/C âgées d'environ 6 semaines sont utilisées dans toutes les expériences. Chaque souris reçoit une injection sous-cutanée avec $10^6$ cellules M109 dans le flanc droit. Quand les tumeurs atteignent une taille d'environ 300 $mm^3$, les souris reçoivent 200 µl de complexes ADN/lipides contenant 50 µg d'ADN, par injection intraveineuse. Les tissus sont prélevés 24 heures après injection et stockés à -70°C jusqu'à utilisation.

4) Essai CAT

Les tissus sont homogénéisés en utilisant un appareil « FastPrep Cell Disrupter FP120 » (Bio 101/Savant). Les échantillons sont alors centrifugés à 1000 tours/minute pendant 5 minutes. La quantité de transgène CAT exprimé est déterminée en utilisant une procédure standard CAT ELISA (Roche, IN).

5) Résultats

La courbe dose-réponse du composé D sur l'activité de transfert de gène de complexes ADN/lipide cationique/DOPE a été étudiée. Le composé « Analogue D » à été utilisé comme contrôle négatif. Comme prévu, le transfert de gène le plus important a lieu dans les poumons. Il a été observé que l'utilisation du composé « Analogue D » inhibe l'activité transfectante dans les poumons et les tumeurs de façon dose-dépendante. En augmentant la quantité de composé D , l'activité transfectante est également inhibée dans les poumons. Par contre, le transfert de gène ne semble pas être affecté dans les tumeurs. Ces résultats sont conformes à l'hypothèse que l'environnement acide des tumeurs conduit à une séparation des parties PEG du reste de la particule après extravasation.

Les résultats obtenus et présentés à la figure 7 mettent ainsi en évidence que le composé D permet d'augmenter l'activité de transfert de gène spécifique des tumeurs par rapport aux poumons d'un facteur 40.

**EXEMPLE 12 : Etude de la stabilité des composés acidosensibles en fonction du pH.**

Cet exemple illustre le fait que les composés acidosensibles selon la présente invention ont une acidosensibilité modulable selon la nature du cycle orthoester présent (cycle à 5 ou 6 chaînons).

A cet effet, on mesure l'évolution de la taille de complexes nucléolipidiqusiques (identiques à ceux utilisés aux exemples 9 et 10) en fonction du pH et du temps, pour différents lipides pegoylés acidosensibles, ce qui permet de balayer différentes gammes de sensibilité. Ces études sont réalisées à des rapports lipide cationique témoin/ADN et lipide pegoylé acidosensible/ADN fixés.

On prépare des échantillons de 1 ml de complexe ADN/lipide cationique témoin/lipide pegoylé acidosensible tels que :

- le rapport lipide cationique témoin/ADN est de 1,5 nmol/µg,
- le rapport lipide pegoylé acidosensible/ADN est de 0,5 ou 1, et
- la concentration en ADN est de 20 µg/ml dans 75 mM d'une solution de chlorure de sodium.

Après 30 minutes, 500 µl d'une solution tampon à 0,05 mol/l et 500 µl d'une solution de chlorure de sodium à 150 mM sont ajoutés à ces échantillons thermostatés à 37°C dans une étuve ventilée. On établit ainsi un pH acide et la taille des particules est mesurée en fonction du temps.

La concentration finale des échantillons est de 10 µg d'ADN/ml dans 75 mM d'une solution de chlorure de sodium. Les tampons utilisés sont des tampons acide citrique/citrate de sodium à pH 4, pH 5 et pH 6 et un tampon Hepes/soude à pH 7,4.

Les résultats obtenus sont représentés à la figure 5 qui représente l'évolution de la taille des particules nucléolipidiques en fonction du pH et du temps pour les composés acidosensibles C et E préparés aux exemples précédents, et qui ne diffèrent que par la nature du cycle orthoester utilisé (cycle à 5 ou 6 chaînons).

Pour ces deux composés, on observe une augmentation de la taille des particules nucléolipidiques en fonction du temps lorsque le pH est acide, ce qui témoigne de leur dégradation. En revanche, une augmentation de la taille des particules nucléolipidiques en fonction du temps n'est pas observée lorsque le pH est de 7,4. En outre, l'agrégation

des particules nucléolipidiques est d'autant plus rapide que le pH est faible.
Enfin, une grande différence de cinétique entre les deux composés C et E testés est observée : à pH 6, l'agrégation des particules nucléolipidiques commence environ 1 heure après l'acidification dans le cas de l'utilisation du composé E alors que l'utilisation du composé C permet une stabilisation pendant au moins 4 heures.

Ces résultats mettent ainsi en évidence plusieurs propriétés remarquables des composés acidosensibles C et E, et plus généralement des composés acidosensibles de ce type selon la présente demande :

- ils présentent tous deux une sensibilité importante aux pH acides, et dans tous les cas, on observe que leur déstabilisation est d'autant plus rapide que le pH est faible,
- ils sont tous deux relativement stables à pH physiologique (pH de 7,4), et
- leur cinétique de dégradation est très différente selon le groupe orthoester utilisé (cycle à 5 ou à 6 chaînons).

**Revendications**

1. Composés acidosensibles **caractérisés en ce qu'**ils ont pour formule générale :

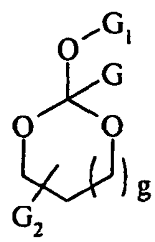

(I)

dans laquelle :

• g est un entier pouvant prendre les valeurs 0, 1, 2, 3 ou 4,
• G représente un atome d'hydrogène, un radical alkyl contenant 1 à 6 atomes de carbone, saturé ou insaturé, en chaîne droite ou ramifiée, ou un radical aryle contenant 6 à 14 atomes de carbone,
• $G_1$ et $G_2$ représentent :

(a) l'un un substituant hydrophile choisi parmi les radicaux de type polyamine, et l'autre un substituant hydrophobe choisi parmi les alkyles mono- ou bicaténaires, les dérivés de stéroïde ou les dendrimères hydrophobes, ou bien
(b) l'un un groupe alkyle linéaire hydrophobe comprenant 10 à 24 atomes de carbones et comprenant éventuellement une ou plusieurs insaturations, et l'autre un groupe de formule générale :

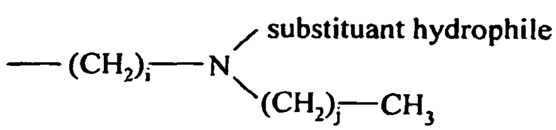

(II)

dans laquelle i est un entier allant de 1 à 4 et j est un entier allant de 9 à 23, et le substituant hydrophile est choisi parmi les radicaux de type polyamine, ou bien
(c) l'un un substituant hydrophile choisi parmi les polyalkylène glycols ou les mono- ou polysaccharides et l'autre un substituant choisi parmi les polyalkylène imines, ou bien
(d) l'un un substituant hydrophile choisi parmi les polyalkylène glycols ou les mono- ou polysaccharides et l'autre un substituant hydrophobe choisi parmi les alkyles mono- ou bicaténaires, les dérivés de stéroïde, les dendrimères hydrophobes, ou les conjugués covalents entre un alkyle mono- ou bicaténaire, un dérivé de stéroïde, ou un dendrimère hydrophobe et une molécule de polyalkylène glycol comprenant 1 à 20 unités monomériques, ou bien
(e) l'un un substituant hydrophile choisi parmi les polyalkylène glycols ou les mono- ou polysaccharides et l'autre une molécule thérapeutique, ou bien
(f) l'un une molécule thérapeutique de nature hydrophile et l'autre un substituant hydrophobe choisi parmi les alkyles mono- ou bicaténaires, les dérivés de stéroïde ou les dendrimères hydrophobes, ainsi que

leurs sels.

2. Composés acidosensibles selon la revendication 1 **caractérisés en ce que** G est choisi parmi l'hydrogène, le méthyle, l'éthyle ou le phényle.

3. Composés acidosensibles selon la revendication 1 **caractérisés en ce que** les alkyles mono- ou bicaténaires sont constitués d'une ou deux chaînes alkyles linéaires comprenant 10 à 24 atomes de carbones et comprenant éventuellement une ou plusieurs insaturations.

4. Composés acidosensibles selon la revendication 1 **caractérisés en ce que** le dérivé de stéroïde est choisi parmi les stérols, les stéroïdes et les hormones stéroïdiennes.

5. Composés acidosensibles selon la revendication 1 **caractérisés en ce que** le dendrimère hydrophobe est le poly (benzyl éther).

6. Composés acidosensibles selon la revendication 1 **caractérisés en ce que** les polyalkylène glycols sont choisis parmi les polyalkylènes glycols de poids moléculaire moyen compris entre $10^2$ et $10^5$ Daltons.

7. Composés acidosensibles selon la revendication 6 **caractérisés en ce que** les polyalkylène glycols sont choisis parmi les polyéthylène glycols (PEG) de poids moléculaire moyen compris entre $10^2$ et $10^5$ Daltons.

8. Composés acidosensibles selon la revendication 1 **caractérisés en ce que** les mono- ou poly-saccharides sont choisis parmi les pyranoses, les furanoses, les dextrans, l'$\alpha$-amylose, l'amylopectine, les fructans, les mannans, les xylans et les arabinans.

9. Composés acidosensibles selon la revendication 1 **caractérisés en ce que** le polyalkylène glycol ou le mono- ou polysaccharide est lié covalemment à un élément de ciblage.

10. Composés acidosensibles selon la revendication 9 **caractérisés en ce que** l'élément de ciblage est choisi parmi les sucres, les peptides, les protéines, les oligonucléotides, les lipides, les neuromédiateurs, les hormones, les vitamines ou leurs dérivés.

11. Composés acidosensibles selon la revendication 1 **caractérisés en ce que** les polyalkylèneimines sont choisis parmi les polymères comprenant les unités monomériques de formule générale :

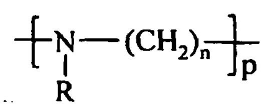

dans laquelle R peut être un atome d'hydrogène ou un groupe de formule :

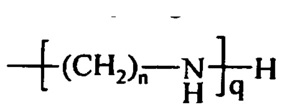

et n est un nombre entier compris entre 2 et 10, p et q sont des nombres entiers choisis de telle sorte que la somme p + q est telle que le poids moléculaire moyen du polymère soit compris entre 100 et $10^7$ Da,
étant entendu que la valeur de n peut varier entre les différents motifs $-NR-(CH_2)n-$.

12. Composés acidosensibles selon la revendication 1 **caractérisés en ce que** chacun des substituant $G_1$ et $G_2$ est indirectement lié à l'orthoester cyclique par l'intermédiaire d'une molécule « espaceur ».

13. Composés acidosensibles selon la revendication 12 **caractérisés en ce que** ladite molécule « espaceur » est choisie parmi les alkyles (1 à 6 atomes de carbone), les liaisons carbonyles, esters, éthers, amide, carbamate, thiocarbamate, le glycérol, l'urée, la thiourée, ou une combinaison de plusieurs de ces groupes.

**14.** Composés acidosensibles selon la revendication 1 **caractérisés en ce que** les molécules thérapeutiques sont choisies parmi les peptides, les oligopeptides, les protéines, les antigènes et leurs anticorps, les enzymes et leurs inhibiteurs, les hormones, les antibiotiques, les analgésiques, les bronchodilatateurs, les antimicrobiens, les agents antihypertenseurs, les agents cardiovasculaires, les agents agissants sur le système nerveux central, les antihistaminiques, les antidépresseurs, les tranquilisants, les anticonvulsifs, les substances anti-inflammatoires, les stimulants, les antiémétiques, les diurétiques, les antispasmodiques, les antiischémiques, les agents limitant la mort cellulaire, ou les agents anticancéreux.

**15.** Compositions **caractérisées en ce qu'**elles comprennent au moins un composé acidosensible tel que défini dans les revendications 1 à 14.

**16.** Compositions **caractérisées en ce qu'**elles comprennent au moins une substance biologiquement active et un composé acidosensible tel que défini à la revendication 1 et pour lequel $G_1$ et $G_2$ ont les définitions indiquées sous (a), (b), (c) ou (d).

**17.** Compositions selon la revendication 16 **caractérisées en ce que** ladite substance biologiquement active est soit une molécule thérapeutique telle que définie à la revendication 15, soit un acide nucléique.

**18.** Compositions selon l'une des revendications 15 ou 16 **caractérisées en ce qu'**elles comprennent en outre un ou plusieurs adjuvants.

**19.** Compositions selon la revendication 18 **caractérisées en ce que** ledit adjuvant est un ou plusieurs lipides neutres.

**20.** Compositions selon la revendication 19 **caractérisées en ce que** ledit adjuvant est choisi parmi les lipides naturels ou synthétiques, zwitterioniques ou dépourvus de charge ionique dans les conditions physiologiques.

**21.** Compositions selon la revendication 20 **caractérisées en ce que** ledit adjuvant est choisi parmi la dioleoylphosphatidyléthanolamine (DOPE), l'oléoylpalmitoyl-phosphatidyléthanolamine (POPE), les di-stéaroyl, -palmitoyl, -mirystoyl phosphatidyléthanolamines ainsi que leurs dérivé N-méthylés 1 à 3 fois, les phosphatidylglycérols, les diacylglycérols, les glycosyldiacylglycérols, les cérébrosides (tels que notamment les galactocérébrosides), les sphingolipides (tels que notamment les sphingomyélines) ou encore les asialogangliosides (tels que notamment les asialoGM1 et GM2).

**22.** Compositions selon l'une des revendications 15 à 21 **caractérisées en ce qu'**elles comprennent en outre un véhicule pharmaceutiquement acceptable pour une formulation injectable.

**23.** Compositions selon l'une des revendications 15 à 21 **caractérisées en ce qu'**elles comprennent en outre un véhicule pharmaceutiquement acceptable pour une administration sur la peau et/ou les muqueuses.

**24.** Utilisation d'un composé acidosensible tel que défini dans les revendications 1 à 14 pour fabriquer un médicament destiné à soigner les maladies.

**25.** Utilisation d'un composé acidosensible tel que défini tel que à la revendication 1 et pour lequel $G_1$ et $G_2$ ont les définitions indiquées sous (a), (b), (c) ou (d), pour fabriquer un médicament destiné à la transfection d'acides nucléiques.

**26.** Les composés acidosensibles tels que définis dans la revendication 1 et pour lesquels $G_1$ et $G_2$ ont les définitions indiquées sous (e) ou (f) pour utilisation comme médicament.

## Patentansprüche

**1.** Säureempfindliche Verbindungen, **dadurch gekennzeichnet, dass** sie die folgende allgemeine Formel aufweisen:

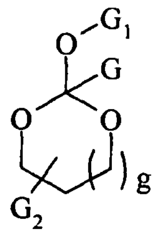 (I)

in der:

- g eine ganze Zahl ist, die die Werte 0, 1, 2, 3 oder 4 annehmen kann,
- G ein Wasserstoffatom, ein 1 bis 6 Kohlenstoffatome enthaltendes, gesättigtes oder ungesättigtes, Alkylradikal in gerader oder verzweigter Kette oder ein 6 bis 14 Kohlenstoffatome enthaltendes Arylradikal darstellt,
- $G_1$ und $G_2$ Folgendes darstellen:

(a) das eine ein aus den Radikalen vom Typ Polyamin ausgewähltes hydrophiles Substituent und das andere ein aus den Mono- oder Bi-Ketten-Alkylen, den steroiden Derivaten oder den hydrophoben Dendrimeren ausgewähltes hydrophobes Substituent, oder
(b) eines eine lineare, hydrophobe Alkylgruppe mit 10 bis 24 Kohlenstoffatomen und eventuell mit einer oder mehreren Unsättigung(en), und die andere eine Gruppe der allgemeinen Formel:

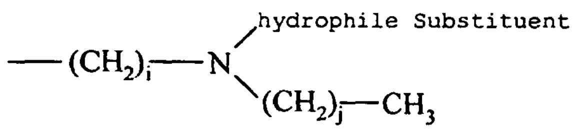 (II)

in der i eine ganze Zahl zwischen 1 und 4 ist und j eine ganze Zahl zwischen 9 und 23 und der hydrophile Substituent aus den Radikalen vom Typ Polyamin ausgewählt wird, oder
(c) eines ein aus den Polyalkylen-Glykolen oder den Mono- oder PolySacchariden ausgewähltes Substituent ist und das andere ein aus den Imin-Polyalkylen ausgewähltes hydrophiles Substituent oder
(d) eines ein aus den Glykol-Polyalkylenen oder den Mono- oder Polysacchariden ausgewähltes hydrophiles Substituent und das andere ein aus den Mono- oder Bi-Ketten-Alkylen, den Steroid-Derivaten, den hydrophoben Dendrimeren oder den kovalenten konjugierten Doppelbindungen zwischen einem Mono- oder Bi-Ketten-Alkyl ausgewähltes hydrophobes Substituent ist, oder ein hydrophobes Dendrimer und ein Glykol-Polyalkylen-Molekül mit 1 bis 20 monomerischen Einheiten, oder
(e) eines ein aus den Glykol-Polyalkylenen oder den Mono- oder Polysacchariden ausgewähltes hydrophiles Substituent und das andere ein therapeutisches Molekül ist oder
(f) eines ein therapeutisches Molekül hydrophiler Art und das andere ein aus den Mono- oder Bi-Ketten-Alkylen, den Steroid-Derivaten oder den hydrophoben Dendrimeren ausgewähltes hydrophobes Substituent ist, sowie deren Salze.

2. Säureempfindliche Verbindungen gemäß Anspruch 1, **dadurch gekennzeichnet, dass** G aus Wasserstoff, Methyl, Ethyl oder Phenyl ausgewählt ist.

3. Säureempfindliche Verbindungen gemäß Anspruch 1, **dadurch gekennzeichnet; dass** die Mono- oder Bi-Ketten-Alkyle aus einer oder zwei linearen Alkylketten mit 10 bis 24 Kohlenstoffatomen und eventuell einer oder mehreren Unsättigung(en) gebildet werden.

4. Säureempfindliche Verbindungen gemäß Anspruch 1, **dadurch gekennzeichnet, dass** das Steroid-Derivat aus den Sterolen, den Steroiden und steroidischen Hormonen ausgewählt wird.

5. Säureempfindliche Verbindungen gemäß Anspruch 1, **dadurch gekennzeichnet, dass** das hydrophobe Dendrimer das Poly(Benzyl-Ester) ist.

6. Säureempfindliche Verbindungen gemäß Anspruch 1, **dadurch gekennzeichnet, dass** die Glykol-Polyalkylene

aus den Glykol-Alkylenen mit einem zwischen $10^2$ und $10^5$ Dalton inbegriffenen durchschnittlichen Molekulargewicht ausgewählt werden.

**7.** Säureempfindliche Verbindungen gemäß Anspruch 6, **dadurch gekennzeichnet, dass** die Glykol-Polyalkylene aus den Glykol-Polyehtylenen (PEG) mit einem zwischen $10^2$ und $10^5$ Dalton inbegriffenen durchschnittlichen Molekulargewicht ausgewählt werden.

**8.** Säureempfindliche Verbindungen gemäß Anspruch 1, **dadurch gekennzeichnet, dass** die Mono- oder Poly-Saccharide aus den Pyranosen, den Furanosen, den Dextranen, der α-Amylose, dem Amylopectin, dem Fruktan, den Mannanen, den Xylanen und den Arabinen ausgewählt werden.

**9.** Säureempfindliche Verbindungen gemäß Anspruch 1, **dadurch gekennzeichnet, dass** das Glykol-Polyalykylen oder das Mono- oder Poly-Saccharid kovalent mit einem Targeting-Element verbunden ist.

**10.** Säureempfindliche Verbindungen gemäß Anspruch 9, **dadurch gekennzeichnet**, das das Targeting-Element aus den Zuckern, den Peptiden, den Proteinen, den Oligonukleotiden, den Lipiden, den Neuromediatoren, den Hormonen, den Vitaminen oder ihren Derivaten ausgewählt wird.

**11.** Säureempfindliche Verbindungen gemäß Anspruch 1, **dadurch gekennzeichnet, dass** die Imin-Polyalkylen aus den Polymeren mit den monomerischen Einheiten der folgenden allgemeinen Formel ausgewählt werden:

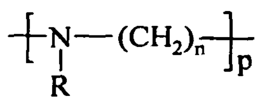

in der R ein Wasserstoffatom oder eine Gruppe mit der folgenden Formel sein kann:

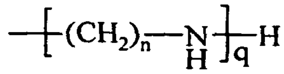

und n eine zwischen 2 und 10 inbegriffene ganze Zahl ist, p und q zwei derart ausgewählte ganze Zahlen sind, dass die Summe p + q derart ist, dass das durchschnittliche Molekulargewicht des Polymers zwischen 100 und $10^7$ Da inbegriffen ist,
wobei es sich von selbst versteht, dass der Wert von n zwischen den unterschiedlichen Motiven -NR-$(CH_2)$n- variieren kann.

**12.** Säureempfindliche Verbindungen gemäß Anspruch 1, **dadurch gekennzeichnet, dass** jedes Substituent $G_1$ und $G_2$ mittels eines "Platzhalter"-Moleküls indirekt mit dem zyklischen Orthoester verbunden ist.

**13.** Säureempfindliche Verbindungen gemäß Anspruch 12, **dadurch gekennzeichnet, dass** das genannte "Platzhalter"-Molekül aus den Alkylen (1 bis 6 Kohlenstoffatome), den Karbonyl-, Ester-, Äther-, Amid-, Karabamat-, Thiokarbamat-, Glycerin-, Harnstoff-, Thioharnstoffverbindungenoder einer Verbindung mehrerer dieser Gruppen ausgewählt wird.

**14.** Säureempfindliche Verbindungen gemäß Anspruch 1, **dadurch gekennzeichnet, dass** die therapeutischen Moleküle aus den Peptiden, den Oligopeptiden, den Proteinen, den Antigenen und ihren Antikörpern, den Enzymen und ihren Inhibitoren, den Hormonen, den Antibiotika, den Analgetika, den Bronchodilatatoren, den antimikrobiellen Wirkstoffen, den blutdrucksenkenden Wirkstoffen, den Herz-Kreislauf-Wirkstoffen, den auf das zentrale Nervensystem einwirkenden Wirkstoffen, den Antihistaminen, den Antidepressiva, den Beruhigungsmitteln, den Antikonvulsiva, den anti-inflammatorischen Substanzen, den Stimulantien, den Antiemetica, den Diuretika, den Antispasmodika, den anti-ischämischen Wirkstoffen den den Zelltod begrenzenden Wirkstoffen oder den Anti-Krebs-Wirkstoffen ausgewählt werden.

**15.** Zusammensetzungen, **dadurch gekennzeichnet, dass** sie wenigstens eine säureempfindliche Verbindung gemäß Definition in den Ansprüchen 1 bis 14 umfassen.

16. Zusammensetzungen, **dadurch gekennzeichnet, dass** sie wenigstens eine biologisch aktive Substanz und eine säureempfindliche Verbindung gemäß Definition in Anspruch 1 umfassen und bei denen $G_1$ und $G_2$ gemäß (a), (b), (c) oder (d) definiert werden.

17. Zusammensetzungen gemäß Anspruch 16, **dadurch gekennzeichnet, dass** die genannte aktive biologische Substanz entweder ein therapeutisches Molekül gemäß Definition in Anspruch 15 ist oder eine Nukleinsäure.

18. Zusammensetzungen gemäß Anspruch 15 oder 16, **dadurch gekennzeichnet, dass** sie darüber hinaus einen oder mehrere Zusatzstoff(e) umfassen.

19. Zusammensetzungen gemäß Anspruch 18, **dadurch gekennzeichnet, dass** der genannte Zusatzstoff ein oder mehrere neutrale(s) Lipid(e) ist.

20. Zusammensetzungen gemäß Anspruch 19, **dadurch gekennzeichnet, dass** der genannte Zusatzstoff aus den natürlichen oder synthetischen, zwitterionischen oder unter physiologischen Bedingungen keine Ionenladungen enthaltenden Lipiden ausgewählt wird.

21. Zusammensetzungen gemäß Anspruch 20, **dadurch gekennzeichnet, dass** der genannte Zusatzstoff aus dem Dioleoylphosphatidylethanolamin (DOPE), dem Oleoylpalmitoyl-Phosphatidylethanolamin (POPE), den Di-Stearoyl, -Palmitoyl, -Mirystoyl-Phosphatidylethanolaminen sowie ihren 1 bis 3 Mal N-methylierten Derivaten, den Phosphatidylglycerinen, den Diacylglycerinen, den Glycosyldiacylglycerinen, den Cerebrosiden (wie z. B. insbesondere den Galactocerebrosiden), den Sphingolipiden (wie z. B. insbesondere den Sphingomyelinen) oder auch den Asialogangliosiden (wie z. B. insbesondere den AsialoGM1 und GM2) ausgewählt w3rden.

22. Zusammensetzungen gemäß Anspruch 15 bis 21, **dadurch gekennzeichnet, dass** sie darüber hinaus einen für eine injizierbre Formulierung pharmazeutisch akzeptablen Träger umfassen.

23. Verbindungen gemäß Anspruch 15 bis 21, **dadurch gekennzeichnet, dass** sie darüber hinaus einen für eine Verabreichung auf der Haut und / oder den Schleimhäuten akzeptablen pharmazeutischen Träger umfassen.

24. Einsatz einer säureempfindlichen Verbindung gemäß Definition in den Ansprüchen 1 bis 14 zur Herstellung eines zur Behandlung von Krankheiten bestimmten Medikaments.

25. Herstellung einer säureempfindlichen Verbindung gemäß Definition in Anspruch 1 und bei der $G_1$ und $G_2$ gemäß (a), (b), (c) oder (d) definiert sind, zur Herstellung eines zur Transfektion von Nukleinsäuren bestimmten Medikaments.

26. Die säureempfindlichen Verbindungen gemäß Anspruch 1, bei denen $G_1$ und $G_2$ die unter (e) oder (f) angegebenen Definitionen haben, zu Verwendung als Medikament.

## Claims

1. Acid-sensitive compounds **characterised in that** they have the following general formula :

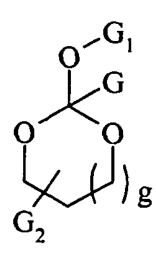

(I)

in which :

- g is a whole number that can have the value of 0, 1, 2, 3 or 4,
- G represents a hydrogen atom, an alkyl radical containing 1 to 6 saturated or unsaturated carbon atoms, in a straight or branched chain, or an aryl radical containing 6 to 14 carbon atoms,
- $G_1$ and $G_2$ represent :

(a) one a hydrophilic substituent chosen from among the polyamine type radicals, and the other a hydrophobic substituent chosen from among the mono- or bicatenary alkyls, the steroid derivatives or the hydrophobic dendrimers, or even
(b) one a hydrophobic linear alkyl group comprising 10 to 24 carbon atoms and eventually comprising one or several unsaturations, and the other a group with the general formula:

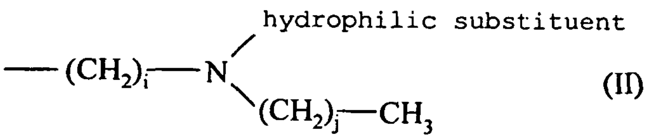

(II)

in which i is a whole number ranging from 1 to 4 and j is a whole number ranging from 9 to 23, and the hydrophilic substituent is chosen from among the polyamine type radicals, or even
(c) one a hydrophilic substituent chosen from among the polyalkylene glycols or the mono- or polysaccharides and the other a substituent chosen from among the polalkylene imines, or even
(d) one a hydrophilic substituent chosen from among the polyalkylene glycols or the mono- or polysaccharides and the other a hydrophobic substituent chosen from among the mono- or bicatenary alkyls, the steroid derivatives, the hydrophobic dendrimers, or the covalent conjugates between a mono- or bicatenary alkyl, a steroid derivative, or a hydrophobic dendrimer and a polyalkylene glycol molecule comprising 1 to 20 monomeric units, or even
(e) one a hydrophilic substituent chosen from among the polyalkylene glycols or the mono- or polysaccharides and the other a therapeutic molecule, or even
(f) one a therapeutic molecule of the hydrophilic type and the other a hydrophobic substituent chosen from among the mono- or bicatenary alkyls, the steroid derivatives or the hydrophobic dendrimers, as well as their salts.

**2.** Acid-sensitive compounds according to claim 1 **characterised in that** G is chosen from among hydrogen, methyl, ethyl or phenyl.

**3.** Acid-sensitive compounds according to claim 1 **characterised in that** the mono- or bicatenary alkyls consist of one or two linear alkyl chains comprising 10 to 24 carbon atoms and eventually comprising one or several unsaturations.

**4.** Acid-sensitive compounds according to claim 1 **characterised in that** the steroid derivative is chosen from among the sterols, steroids and steroid hormones.

**5.** Acid-sensitive compounds according to claim 1 **characterised in that** the hydrophobic dendrimer is poly(benzyl ether).

**6.** Acid-sensitive compounds according to claim 1 **characterised in that** the polyalkylene glycols are chosen from among the polyalkylene glycols of mean molecular weight ranging from $10^2$ to $10^5$ Daltons.

**7.** Acid-sensitive compounds according to claim 6 **characterised in that** the polyalkylene glycols are chosen from among the polyethylene glycols (PEG) of mean molecular weight ranging from $10^2$ to $10^5$ Daltons.

**8.** Acid-sensitive compounds according to claim 1 **characterised in that** the mono- or poly-saccharides are chosen from among the pyranoses, furanoses, dextrans, α-amylose, amylopectin, fructans, mannans, xylans and arabinans.

**9.** Acid-sensitive compounds according to claim 1 **characterised in that** the polyalkylene glycol or mono- or polysaccharide is covalently bonded to a targeting element.

**10.** Acid-sensitive compounds according to claim 9 **characterised in that** the targeting element is chosen from among the sugars, peptides, proteins, oligonucleotides, lipids, neuromediators, hormones, vitamins or their derivatives.

**11.** Acid-sensitive compounds according to claim 1 **characterised in that** the polyalkyleneimines are chosen from among the polymers comprising monomeric units with the following genera formula :

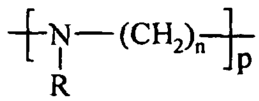

n which R may be a hydrogen atom or a group with the following formula:

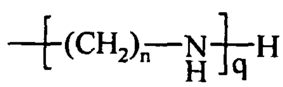

and n is a whole number ranging from 2 to 10, p and q are whole numbers chosen so that the sum of p + q is such that the mean molecular weight of the polymer ranges from 100 to $10^7$ Da,
knowing that the value of n can vary between the different units $-NR-(CH_2)n-$.

**12.** Acid-sensitive compounds according to claim 1 **characterised in that** each substituent $G_1$ and $G_2$ is indirectly bonded to the cyclic orthoester by means of a « spacing » molecule.

**13.** Acid-sensitive compounds according to claim 12 **characterised in that** the so-called « spacing » molecule is chosen from among the alkyls (1 to 6 carbon atoms), carbonyl bonds, esters, ethers, amide, carbamate, thiocarbamate, glycerol, urea, thiourea, or a combination of several of these groups.

**14.** Acid-sensitive compounds according to claim 1 **characterised in that** the therapeutic molecules are chosen from among the peptides, oligopeptides, proteins, antigens and their antibodies, enzymes and their inhibitors, hormones, antibiotics, analgesics, bronchodilators, anti-microbial drugs, anti-hypertensive agents, cardiovascular agents, agents acting on the central nervous system, antihistaminics, anti-depressors, tranquillisers, anti-convulsive drugs, antiinflammatory substances, stimulants, anti-emetics, diuretics, antispasmodics, anti-ischaemic agents, agents limiting cell death, or anti-cancer agents.

**15.** Compositions **characterised in that** they include at lease one acid-sensitive compound as defined in claims 1 to 14.

**16.** Compositions **characterised in that** they include at lease one biologically active substance and an acid-sensitive compound as defined in claim 1 and in which the definitions of $G_1$ and $G_2$ are indicated under (a), (b), (c) or (d).

**17.** Compositions according to claim 16 **characterised in that** the so-called biologically active substance is either a therapeutic molecule as defined in claim 15, or a nucleic acid.

**18.** Compositions according to claims 15 or 16 **characterised in that** they also comprise one or several additives.

**19.** Compositions according to claim 18 **characterised in that** the aforementioned additive is one or several neutral lipids.

**20.** Compositions according to claim 19 **characterised in that** the aforementioned additive is chosen from among the natural or synthetic lipids, zwitterionics or void of ionic charge in the physiological conditions.

**21.** Compositions according to claim 20 **characterised in that** the aforementioned additive is chosen from among dioleoylphosphatidylethanolamine (DOPE), oleoylpalmitoylphosphatidylethanolamine (POPE), di-stearoyl, -palmitoyl, -mirystoyl phosphatidylethanolamines as well as their 1 to 3 times N-methylated derivative, phosphatidylglycerols, diacylglycerols, glycosyldiacylglycerols, cerebrosides (such as the galactocerebrosides, in particular), the sphingolipids (such as the sphingomyelines, in particular) or even the asialogangliosides (such as the asialoGM1 and GM2, in particular).

**22.** Compositions according to any of claims 15 to 21 **characterised in that** they also include a pharmaceutically acceptable excipient for an injectable formulation.

**23.** Compositions according to any of claims 15 to 21 **characterised in that** they also include a pharmaceutically acceptable excipient for administration on the skin and/or mucous membranes.

**24.** Use of a acid-sensitive compound as defined in claims 1 to 14 to manufacture a drug intended to treat diseases.

**25.** Use of an acid-sensitive compound as defined as in claim 1 and in which the definitions of $G_1$ and $G_2$ are indicated under (a), (b), (c) or (d), to manufacture a drug intended for the transfection of nucleic acids.

**26.** The acid-sensitive compounds as defined in claim 1 and in which the definitions of $G_1$ and $G_2$ are indicated under (e) or (f) for use as a drug.

# FIG. 1

Etude de la libération de l'ADN à pH 5,0

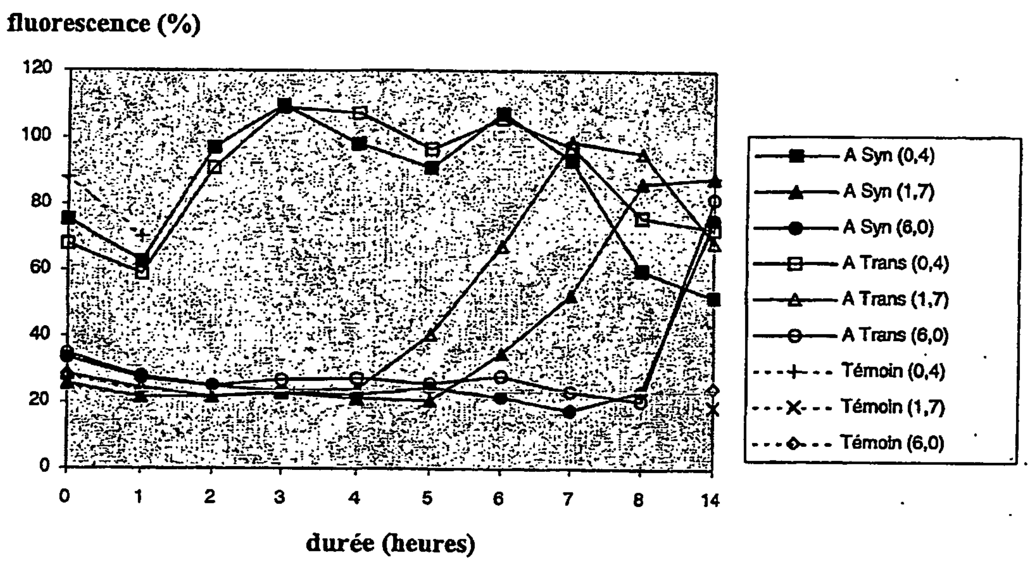

**FIG. 2**

**Efficacité de transfection *in vitro***

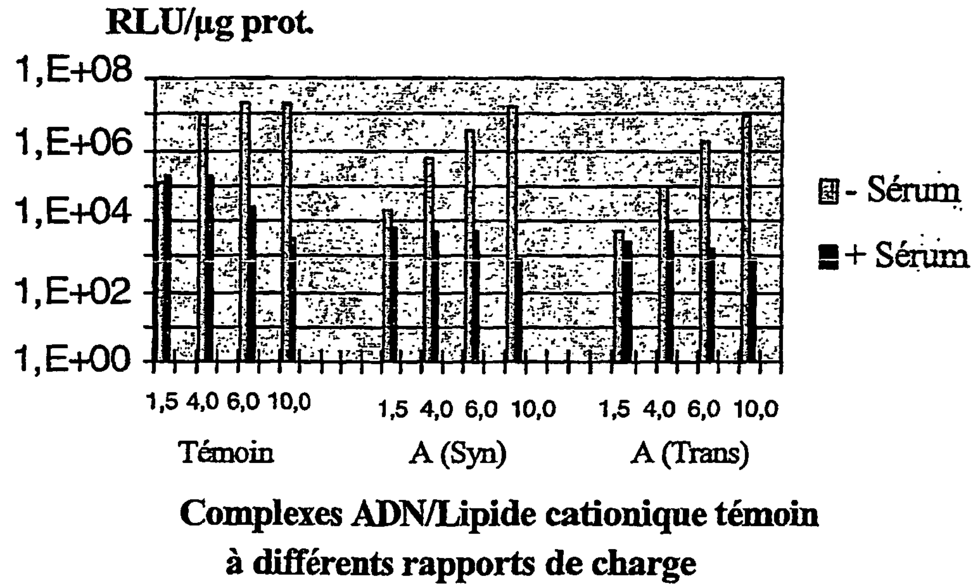

# FIG. 3

**Stabilisation des complexes nucléolipidiques par le composé C, le composé D, le BRIJ700 ou l'analogue D**

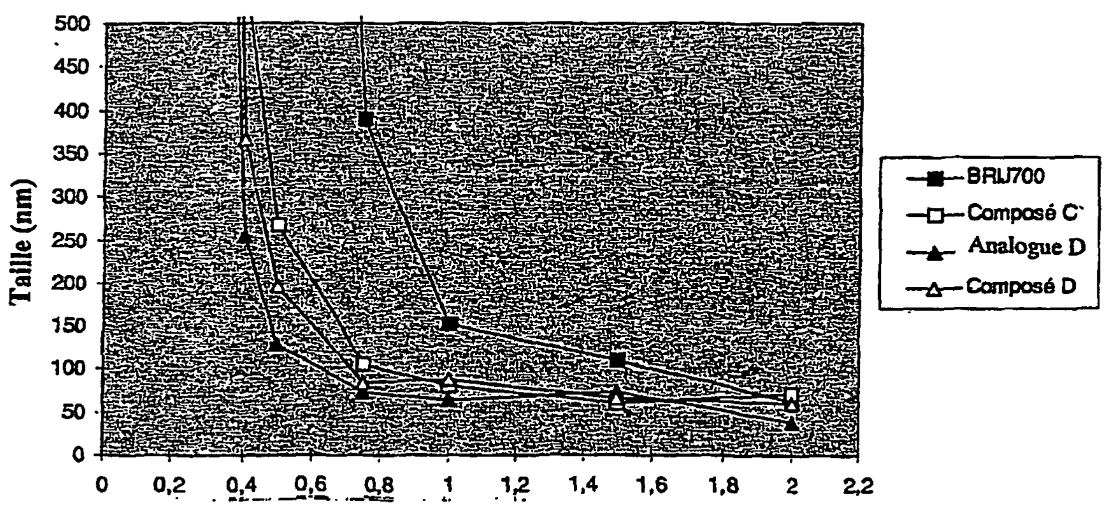


**Ratio poids/poids avec l'ADN**

FIG. 4

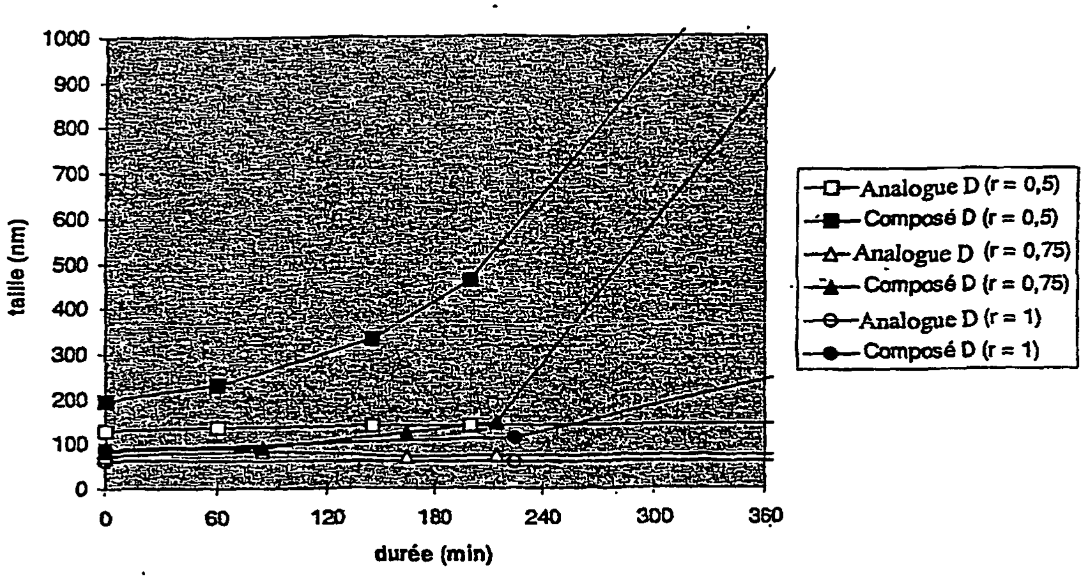
1000
900
800
700
600
500
400
300
200
100
0
taille (nm)
0
60
120
180
240
300
360
durée (min)
Analogue D (r = 0,5)
Composé D (r = 0,5)
Analogue D (r = 0,75)
Composé D (r = 0,75)
Analogue D (r = 1)
Composé D (r = 1)

**FIG. 5**

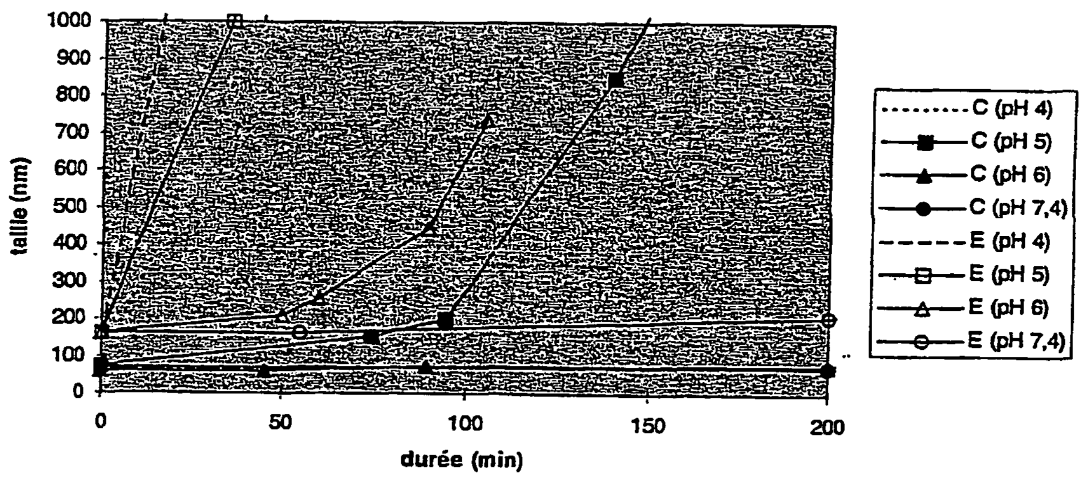
1000
900
800
700
600
500
400
300
200
100
0
taille (nm)
0
50
100
150
200
durée (min)
C (pH 4)
C (pH 5)
C (pH 6)
C (pH 7,4)
E (pH 4)
E (pH 5)
E (pH 6)
E (pH 7,4)

**FIG. 6**

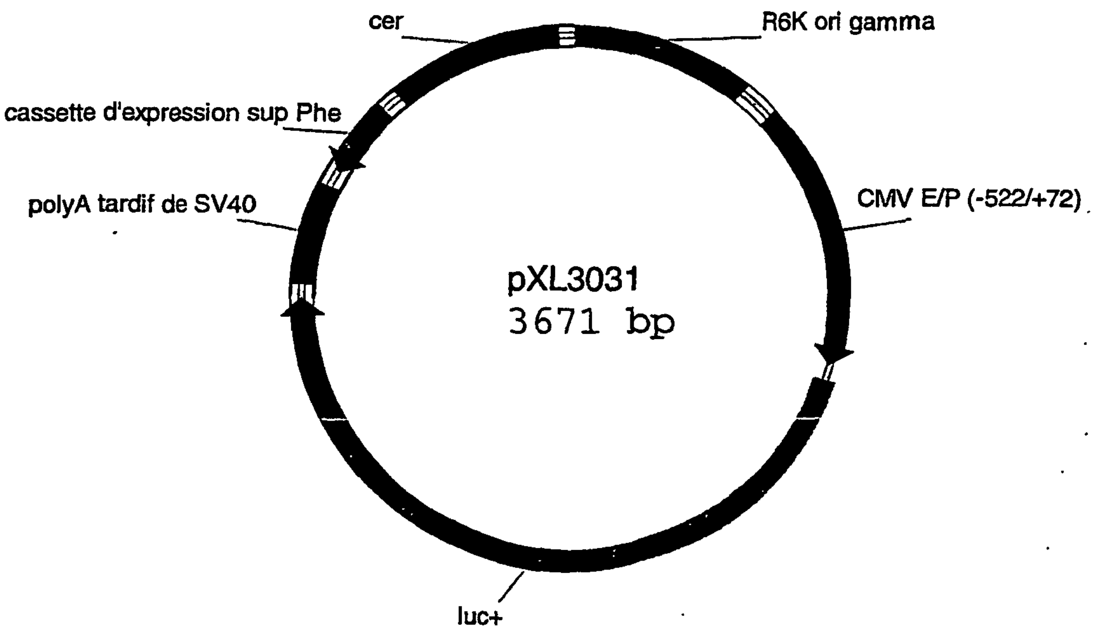
cer
R6K ori gamma
cassette d'expression sup Phe
polyA tardif de SV40
CMV E/P (-522/+72)
pXL3031
3671 bp
luc+

FIG. 7

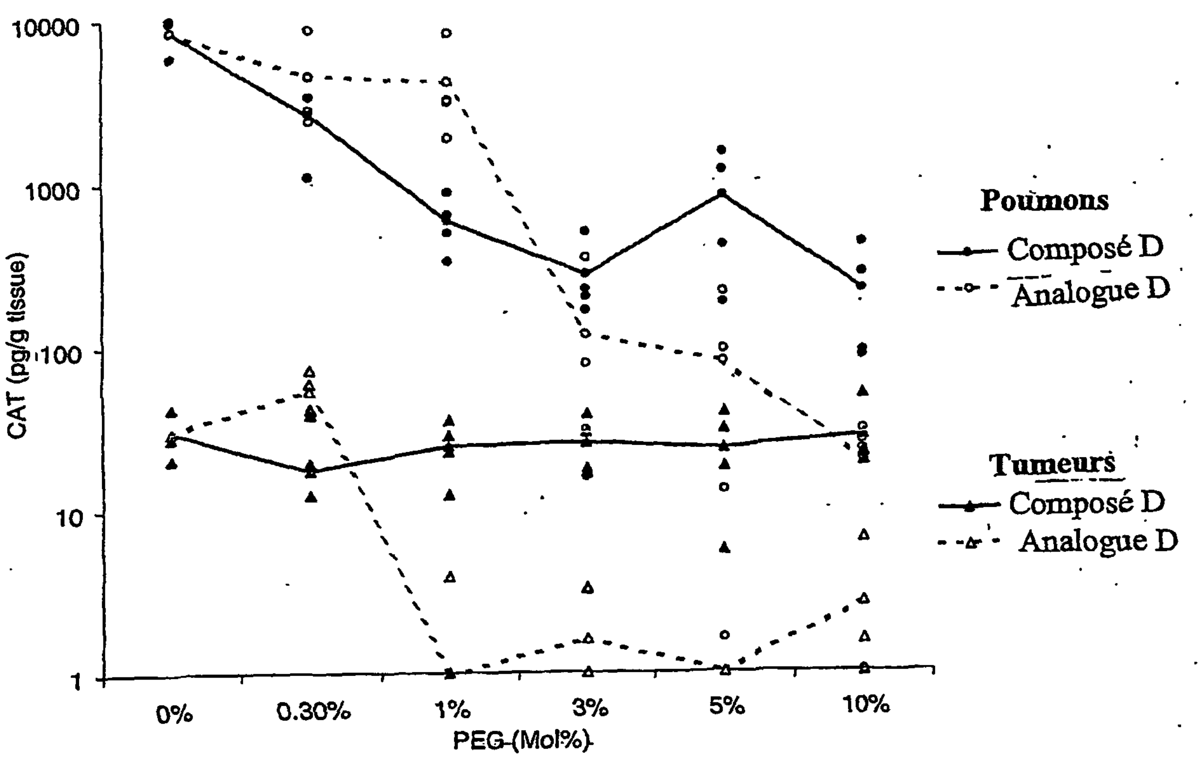
10000
1000
100
10
1
CAT (pg/g tissue)
0%
0.30%
1%
3%
5%
10%
PEG-(Mol%)
Poumons
Composé D
Analogue D
Tumeurs
Composé D
Analogue D